(19) Europäisches Patentamt
European Patent Office
Office européen des brevets

(11) EP 3 229 769 B1

(12) **EUROPÄISCHE PATENTSCHRIFT**

(45) Veröffentlichungstag und Bekanntmachung des Hinweises auf die Patenterteilung:
**23.10.2019 Patentblatt 2019/43**

(21) Anmeldenummer: **15790931.8**

(22) Anmeldetag: **05.11.2015**

(51) Int Cl.:
***A61K 8/46*** (2006.01) ***A61Q 5/08*** (2006.01)
***A61Q 5/10*** (2006.01) ***A61K 8/41*** (2006.01)

(86) Internationale Anmeldenummer:
**PCT/EP2015/075785**

(87) Internationale Veröffentlichungsnummer:
**WO 2016/091492 (16.06.2016 Gazette 2016/24)**

(54) **VERBESSERTE ENTFÄRBUNG VON GEFÄRBTEN KERATINISCHEN FASERN**

BETTER DECOLORATION OF KERATINIC FIBERS

DÉCOLORATION AMÉLIORÉE POUR DES FIBRES KÉRATINIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.12.2014 DE 102014225545**

(43) Veröffentlichungstag der Anmeldung:
**18.10.2017 Patentblatt 2017/42**

(73) Patentinhaber: **Henkel AG & Co. KGaA**
**40589 Düsseldorf (DE)**

(72) Erfinder:
- **SCHÖPGENS, Jürgen**
**41366 Schwalmtal (DE)**
- **MÜLLER, Burkhard**
**40221 Düsseldorf (DE)**

(56) Entgegenhaltungen:
**WO-A2-2007/107310**
**DE-A1- 19 629 453**
**DE-A1-102006 022 274**
**DE-A1-102006 053 343**
**DE-A1-102006 053 402**

- **"Colorants and Finishing Products", , 1. Januar 2000 (2000-01-01), XP055232024, Gefunden im Internet: URL:http://www2.basf.us/pc_textiles/pdfs/CyclanonECO.pdf [gefunden am 2015-11-27]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 229 769 B1

## Beschreibung

Die Erfindung liegt auf dem Gebiet der Kosmetik und betrifft ein Verfahren zur Färbung und zum reduktiven Farbabzug von gefärbten keratinischen Fasern, insbesondere menschlichen Haaren, mit Zusammensetzungen, die in einem kosmetischen Träger mindestens ein Sulfinsäurederivat einer Formel (I) sowie mindestens ein Sulfonsäurederivat einer Formel (II) enthalten. Weiterhin Gegenstand der Erfindung sind auch Mehr-Komponenten-Verpackungseinheiten (Kit-of-parts), mittels welcher sich die zuvor beschriebenen Zusammensetzungen herstellen lassen. Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Zur Erzielung spezieller Nuancen werden häufig werden auch Kombinationen von Oxidationsfarbstoffen und direktziehenden Farbstoffen eingesetzt.

Färbemittel auf Basis von Oxidationsfarbstoffen führen zu brillanten und dauerhaften Farbtönen. Sie bedingen allerdings den Einsatz starker Oxidationsmittel, wie beispielsweise WasserstoffperoxidLösungen. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Färbemittel auf der Basis direktziehender Farbstoffe werden häufig für temporäre Färbungen eingesetzt. Bei den direktziehenden Farbstoffen handelt es sich um Farbstoffmoleküle, die direkt auf das Haar aufziehen und keinen oxidativen Prozess zur Ausbildung der Farbe benötigen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Bei all diesen Färbeprozessen kann es vorkommen, dass die Färbung aus verschiedenen Gründen ganz oder teilweise wieder rückgängig gemacht werden soll. Eine teilweise Entfernung der Färbung bietet sich beispielsweise an, wenn das Färbeergebnis auf den Fasern dunkler ausfällt als gewünscht.

Andererseits kann auch eine vollständige Entfernung der Färbung in manchen Fällen gewünscht sein. So ist es beispielsweise denkbar, dass die Haare für einen konkreten Anlass in einer bestimmten Nuance gefärbt oder getönt werden sollen und nach einigen Tagen die ursprüngliche Farbe wieder zurückgewonnen werden soll.

Mittel und Verfahren zum Farbabzug sind bereits literaturbekannt. Ein aus dem Stand der Technik hinlänglich bekanntes Verfahren, Färbungen wieder rückgängig zu machen, ist die oxidative Nachbehandlung der gefärbten Haare, beispielsweise mithilfe eines üblichen Blondiermittels. Bei diesem Prozess können die Fasern aber durch den Einsatz starker Oxidationsmittel geschädigt werden.

Ferner wurden auch reduktive Prozesse zum Farbabzug bereits beschrieben. So offenbart beispielsweise die europäische Patentanmeldung EP 1 300 136 A2 Verfahren zur Haarbehandlung, bei welchem die Haare in einem ersten Schritt gefärbt und in einem zweiten Schritt wieder reduktiv entfärbt werden. Hierbei erfolgt die reduktive Entfärbung durch Anwendung von einer Formulierung enthaltend ein Dithionitsalz und ein Tensid. In der WO 2008/055756 A2 wird die reduktive Entfärbung von Keratinfasern mittels eines Gemisches aus einem Reduktionsmittel und einem Absorptionsmittel vorgenommen.

Ein mit "Colorants and Finishing Products" übertiteltes technisches Datenblatt zum Rohstoff Cyclanon Eco offenbart, dass sich Cyclanon Eco sehr gut zur Entfernung von nicht fixierten Farbbestandteilen auf verschiedenen Substraten, beispielsweise Wolle, eignet. DE 196 29 453 A1 beschreibt ein Verfahren zur reduktiven Nachreinigung von polyesterhaltigen Textilien. DE 10 2006 022254 A1, DE 10 2006 053402 A1, WO 2007/107310 A2 und DE 10 2006 053343 A1 beschäftigen sich mit der reduktiven Entfärbung von gefärbten Haaren und schlagen für diesen reduktiven Farbabzug jeweils eine Gruppe von Sulfinsäure-Derivaten vor.

Bei Einsatz von reduktiven Entfärbemitteln findet die Entfärbung durch Reduktion der auf den Keratinfasern bzw. Haaren befindlichen Farbstoffe statt. Durch die Reduktion werden die Farbstoffe in der Regel in ihre reduzierten Leukoformen überführt. Bei diesem Vorgang werden die in den Farbstoffen vorhandenen Doppelbindungen reduziert, das chromophore System der Farbstoffe auf diese Weise unterbrochen und der Farbstoff in eine farblose Form umgewandelt.

Ein generelles Problem bei den aus dem Stand der Technik bekannten reduktiven Entfärbemitteln besteht darin, dass die gefärbten Keratinfasern durch Einsatz des Reduktionsmittels zwar zunächst entfärbt werden können, der Farbabzug jedoch nicht von Dauer ist. Insbesondere bei oxidativ gefärbten Haaren, bei denen die Färbung durch Oxidationsfarbstoffvorprodukte vom Entwickler- und vom Kupplertyp auf dem Haar erzeugt wird, werden Färbungen mit teilweise sehr guten Echtheitseigenschaften erhalten. Bei Anwendung des reduktiven Entfärbemittels werden diese Farbstoffe nun reduktiv in ungefärbte Verbindungen überführt - die aufgrund ähnlich guter Echtheitseigenschaften jedoch nach wie vor auf dem Haar verbleiben.

Nach Abspülen des Reduktionsmittels und unter Einwirkung von Luftsauerstoff können diese reduzierten Formen nun nach und nach wieder zurückoxidiert werden. Bedingt durch diese Rückoxidation findet eine mehr oder weniger stark ausgeprägte Rückfärbung statt. Diese Rückfärbung entspricht in der Regel nicht dem Farbton, in dem die Keratinfasern zuvor gefärbt worden waren, sondern kann beliebig unattraktiv ausfallen und wird vom Anwender des Entfärbemittels daher umso weniger erwünscht.

Aufgabe der vorliegenden Erfindung war daher die Bereitstellung eines Entfärbemittels zur Entfärbung von gefärbten keratinischen Fasern, welches gefärbte Keratinfasern möglichst vollständig entfärbt. Die Entfärbung sollte lang anhaltend sein, und die entfärbten Keratinfasern sollten unter der Einwirkung von Luftsauerstoff keine Rückfärbung, keine Nuancenverschiebung und keine Nachdunklung erleiden. Das Entfärbemittel sollte insbesondere auf den Keratinfasern eine gute Entfärbeleistung zeigen, die zuvor mit oxidativen Färbemitteln auf Basis von Oxidationsfarbstoffvorprodukten vom Entwickler- und vom Kupplertyp gefärbt wurden. Zudem soll das Entfärbemittel toxikologisch unbedenklich sein.

Im Zuge der zu dieser Erfindung führenden Arbeiten hat sich herausgestellt, dass diese Aufgabe durch den Einsatz von Entfärbemitteln gelöst werden kann, die in einem kosmetischen Träger ein oder mehrere Sulfinsäurederivate der nachfolgend beschriebenen Formel (I) enthalten.

Verfahren zur reduktiven Nachbehandlung von Polyester-Textilien mit den Sulfinsäurederivaten der Formel (I) sind beispielsweise aus EP 0 914 516 B1 bekannt. Bei der Nachbehandlung der Polyester-Textilien werden die Sulfinsäure-Derivate der Formel (I) jedoch ausschließlich unter kosmetisch wenig akzeptablen Bedingungen, d.h. bei Temperaturen von 50 - 100 °C, eingesetzt. Es konnte nun gefunden werden, dass diese Sulfinsäurederivate sich auch zur Entfärbung von keratinischen Fasern bzw. menschlichen Haaren eignen, wenn die keratinischen Fasern zuvor mit direktziehenden Farbstoffen und/oder Oxidationsfarbstoffvorprodukten (Entwicklern und Kupplern) gefärbt worden waren. Insbesondere überraschend war in diesem Zusammenhang, dass die Entfärbung der Keratinfasern bereits unter physiologisch akzeptablen Bedingungen, d.h. bei Temperaturen unterhalb von 45 °C, stattfand.

Ein erster Gegenstand der vorliegenden Erfindung ist ein Verfahren zur Färbung und reduktiven Entfärbung von keratinischen Fasern, insbesondere menschlichen Haaren, umfassend die folgenden Schritte in der angegebenen Reihenfolge

(I) Applizierung eines kosmetischen Färbemittels, das mindestens einen direktziehenden Farbstoff und/oder mindestens ein Oxidationsfarbstoffvorprodukt enthält, auf die keratinischen Fasern
(II) Einwirkenlassen des Färbemittels für einen Zeitraum von 5 bis 60 Minuten
(III) Ausspülen des Färbemittels
(IV) Applizierung eines Entfärbemittels auf die keratinischen Fasern,
(V) Einwirkenlassen des Entfärbemittels bei 20 bis 45 °C für einen Zeitraum von 5 bis 60 Minuten,
(VI) Ausspülen des Entfärbemittels,

wobei das Entfärbemittel ein Mittel zur reduktiven Entfärbung von gefärbten keratinischen Fasern, insbesondere menschlichen Haaren, enthaltend in einem kosmetischen Träger

(a) mindestens eine Verbindung der Formel (I)

$$A[(CR^1R^2)SO_2M]_q \qquad (I)$$

in der

| | |
|---|---|
| A | für $N(R^3)_{3-q}$ steht, |
| $R^1$, $R^2$ | unabhängig voneinander für ein Wasserstoffatom oder für eine $C_1$-$C_6$-Alkylgruppe stehen, |
| $R^3$ | für identische oder verschiedene Reste ausgewählt aus der Gruppe aus einem Wasserstoffatom, einer $C_1$-$C_{20}$-Alkylgruppe, einer $C_3$-$C_8$-Cycloalkylgruppe ggf. substituiert durch ein bis drei $C_1$-$C_4$-Alkylreste steht, |
| M | für identische oder verschiedene Reste ausgewählt aus einem Wasserstoffatom oder einem Äquivalent eines Alkali-, Erdalkali- oder Metallions, bevorzugt für Natrium, Kalium, ½ Magnesium, ½ Calcium, ½ Zink, oder für ein Ammoniumion ($NH_4^+$) steht, |
| q | für die Zahlen 2 oder 3 steht, |

(b) mindestens eine Verbindung der Formel (II)

$$A[(CR^1R^2)SO_3M]_q \qquad (II)$$

worin A, $R^1$, $R^2$, $R^3$, M und q dieselbe allgemeine Bedeutung wie in Formel (I) besitzen, wobei die Auswahl dieser

Variablen im konkreten Einzelfall für die Verbindungen der Formeln (I) und (II) nicht gleich sein muss.

Die Anwendung der erfindungsgemäßen Entfärbemittel bewirkt - je nach Wahl der zuvor zur Färbung eingesetzten Farbstoffe - eine nahezu vollständige oder sogar vollständige Entfärbung von zuvor gefärbten Keratinfasern. Vor allem auf zuvor mit Oxidationsfarbstoffen gefärbten Keratinfasern zeigt das erfindungsgemäße Entfärbemittel eine hervorragende Wirkung. Als insbesondere überraschend hat sich herausgestellt, dass diese Entfärbewirkung auch nach dem Ausspülen des Reduktionsmittels noch anhält und dass auch die entfärbten Keratinfasern, die für Stunden bzw. Tage der Einwirkung von Luftsauerstoff ausgesetzt werden, kaum Rückoxidation und kaum Nachdunklung erleiden.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Entfärben von Keratinfasern bzw. menschlichen Haaren geeignet sind, steht prinzipiell einer Verwendung auch auf anderen Gebieten nichts entgegen.

Unter dem Begriff "gefärbte keratinische Fasern" werden Keratinfasern verstanden, die mit herkömmlichen, dem Fachmann bekannten kosmetischen Färbemitteln gefärbt wurden. Insbesondere sind unter den "gefärbten keratinischen Fasern" Fasern zu verstehen, die mit den aus dem Stand der Technik bekannten oxidativen Färbemitteln (Entwicklern und Kupplern) gefärbt wurden. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Die Mittel enthalten die erfindungswesentlichen Inhaltsstoffe jeweils in einem kosmetischen Träger, beispielsweise in einem geeigneten wässrigen oder wässrig-alkoholischen Träger. Zum Zwecke der reduktiven Entfärbung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei Mitteln zum reduktiven Farbabzug von keratinischen Fasern um Cremes, Emulsionen oder um fließfähige Gele.

Als erfindungswesentlichen Inhaltstoffe enthalten die Entfärbemittel mindestens ein Sulfinsäurederivat der Formel (I),

$$A[(CR^1R^2)SO_2M]_q \qquad (I)$$

in der

| | |
|---|---|
| A | für $N(R^3)_{3-q}$ steht, |
| $R^1$, $R^2$ | unabhängig voneinander für ein Wasserstoffatom oder für eine $C_1$-$C_6$-Alkylgruppe stehen, |
| $R^3$ | für identische oder verschiedene Reste ausgewählt aus der Gruppe aus einem Wasserstoffatom, einer $C_1$-$C_{20}$-Alkylgruppe, einer $C_3$-$C_8$-Cycloalkylgruppe ggf. substituiert durch ein bis drei $C_1$-$C_4$-Alkylreste steht, |
| M | für identische oder verschiedene Reste ausgewählt aus einem Wasserstoffatom oder einem Äquivalent eines Alkali-, Erdalkali- oder Metallions, bevorzugt für Natrium, Kalium, ½ Magnesium, ½ Calcium, ½ Zink, oder für ein Ammoniumion ($NH_4^+$) steht, |
| q | für die Zahlen 2 oder 3 steht. |

Die Substituenten $R^1$ bis $R^3$ der Verbindungen der Formel (I) sind nachstehend beispielhaft erläutert: Beispiele für eine $C_1$-$C_6$-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für $C_7$-$C_{20}$-Alkylgruppen sind die n-Octyl, die n-Decyl, die n-Dodecyl, die n-Tetradecyl, die n-Hexadecyl und die n-Octadecylgruppe. Beispielhaft für eine C3-C6-Cycloalkylgruppe können die Cyclopropylgruppe, die Cyclopentylgruppe oder die Cyclohexylgruppe genannt werden.
Der Rest A steht in Formel (I) für eine Gruppierung $N(R^3)_{3-q}$
Mit A gleich $N(R^3)_{3-q}$ handelt es sich hierbei demnach um die Formel $N(R^3)_{3-q}$ $[(CR^1R^2)SO_2M]_q$.

Der Rest q kann für die Zahlen 2 oder 3 stehen.
Wenn q für die Zahl 2 steht, handelt es sich bei dem erfindungsgemäßen Sulfinsäurederivat um eine Verbindung der Formel $N(R^3)_1[(CR^1R^2)SO_2M]_2$.
Wenn q für die Zahl 3 steht, handelt es sich bei dem erfindungsgemäßen Sulfinsäurederivat um eine Verbindung der Formel N $[(CR^1R^2)SO_2M]_3$.
In einer ganz besonders bevorzugten Ausführungsform steht q für die Zahl 3.

In einer besonders bevorzugten Ausführungsform ist ein erfindungsgemäßes Mittel dadurch gekennzeichnet, dass es (a) mindestens eine Verbindung der Formel (I) enthält, in der

q für die Zahl 3 steht.

Die Reste R1 und R2 stehen unabhängig voneinander für ein Wasserstoffatom oder für eine $C_1$-$C_6$-Alkylgruppe. Im Hinblick auf die Lösung der erfindungsgemäßen Aufgabenstellung ist es weiterhin besonders bevorzugt, wenn R1 und R2 unabhängig voneinander für ein Wasserstoffatom oder eine Methylgruppe stehen. Ganz besonders bevorzugt stehen die Reste R1 und R2 beide für ein Wasserstoffatom.

In einer weiteren besonders bevorzugten Ausführungsform ist ein erfindungsgemäßes Mittel dadurch gekennzeichnet, dass es (a) mindestens eine Verbindung der Formel (I) enthält, in der

R1, R2 unabhängig voneinander für ein Wasserstoffatom oder für eine Methylgruppe, bevorzugt für ein Wasserstoffatom, stehen.

Der Rest M steht für identische oder verschiedene Reste ausgewählt aus einem Wasserstoffatom oder einem Äquivalent eines Alkali-, Erdalkali- oder Metallions, bevorzugt für Natrium, Kalium, ½ Magnesium, ½ Calcium, ½ Zink, oder für ein Ammoniumion ($NH_4^+$).

Bei den erfindungsgemäßen Sulfinsäurederivaten handelt es sich um Verbindungen, die jeweils - gebunden an ein Stickstoffatom - mehrere Struktureinheiten -($CR^1R^2$)$SO_2M$ besitzen. Bei diesen Struktureinheiten handelt es sich um die mit den Resten R1 und R2 substituierten Sulfinomethylgruppen, die alternativ auch als Methansulfinsäure-Gruppen bezeichnet werden können. In jeder dieser Struktureinheiten kann die Sulfinsäure entweder in protonierter Form vorliegen, in diesem Fall steht M für ein Wasserstoffatom. Die Sulfinsäure kann jedoch auch in Form ihres Salzes, bevorzugt in Form ihres Natriumsalzes, Kaliumsalzes oder Zinksalzes vorliegen.

In den Fällen, in denen das erfindungsgemäße Sulfinsäurederivat mehrere Struktureinheiten - ($CR^1R^2$)$SO_2M$ trägt, kann auch eine Sulfinsäureeinheit protoniert (mit M gleich Wasserstoff) vorliegen, während eine weitere (oder die weiteren) Sulfinsäureeinheiten in Form ihres Salzes (mit M gleich einem Äquivalent eines Alkali-, Erdalkali-, Metall- oder Ammoniumions) vorliegen. Wenn das bzw. die erfindungsgemäßen Sulfinsäurederivate im (gegebenenfalls wässrigen) kosmetischen Träger gelöst vorliegen, stehen die protonierten Formen der Sulfinsäuren mit den deprotonierten Sulfinsäuren im Gleichgewicht, so dass alle diese Formen von der Erfindung explizit mit umfasst sind.

Besonders bevorzugt steht M für identische oder verschiedene Reste ausgewählt aus einem Wasserstoffatom oder einem Äquivalent eines Alkali-, Erdalkali- oder Metallions aus der Gruppe aus Natrium, Kalium und ½ Zink.

In einer weiteren besonders bevorzugten Ausführungsform ist ein erfindungsgemäßes Mittel dadurch gekennzeichnet, dass es (a) mindestens eine Verbindung der Formel (I) enthält, in der

M für identische oder verschiedene Reste ausgewählt aus einem Wasserstoffatom oder einem Äquivalent eines Alkali-, Erdalkali- oder Metallions aus der Gruppe aus Natrium, Kalium und ½ Zink steht.

Synthesebedingt können bei der Herstellung der Verbindungen der Formel (I) auch mehr oder weniger große Mengenanteile an Verbindungen der Formel (II) entstehen, diese sind ebenfalls im erfindungsgemäßen Mittel enthalten.

Die erfindungsgemäßen Mittel zur reduktiven Entfärbung von gefärbten keratinischen Fasern enthalten daher (b) zusätzlich mindestens eine Verbindung der Formel (II)

$$A[(CR^1R^2)SO_3M]_q \qquad (II)$$

Die Reste A, $R^1$, $R^2$, $R^3$, M und q besitzen dieselbe allgemeine Bedeutung wie in Formel (I), wobei die Auswahl dieser Variablen im konkreten Einzelfall für die Verbindungen der Formeln (I) und (II) nicht gleich sein muss.

Die Substituenten $R^1$ bis $R^3$ der Verbindungen der Formel (II) sind nachstehend beispielhaft erläutert: Beispiele für eine $C_1$-$C_6$-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für $C_7$-$C_{20}$-Alkylgruppen sind die n-Octyl, die n-Decyl, die n-Dodecyl, die n-Tetradecyl, die n-Hexadecyl und die n-Octadecylgruppe. Beispielhaft für eine C3-C6-Cycloalkylgruppe können die Cyclopropylgruppe, die Cyclopentylgruppe oder die Cyclohexylgruppe genannt werden.
Der Rest A steht in Formel (II) für eine Gruppierung $N(R^3)_{3-q}$
Mit A gleich $N(R^3)_{3-q}$ handelt es sich hierbei demnach um die Formel $N(R^3)_{3-q}$ $[(CR^1R^2)SO_3M]_q$.
Der Rest q kann für die Zahlen 2 oder 3 stehen.
Wenn q für die Zahl 2 steht, handelt es sich bei dem erfindungsgemäßen Sulfonsäurederivat um eine Verbindung der Formel $N(R^3)_1[(CR^1R^2)SO_3M]_2$.
Wenn q für die Zahl 3 steht, handelt es sich bei dem erfindungsgemäßen Sulfonsäurederivat um eine Verbindung der Formel $N[(CR^1R^2)SO_3M]_3$.

Der Rest M steht für identische oder verschiedene Reste ausgewählt aus einem Wasserstoffatom oder einem Äquivalent eines Alkali-, Erdalkali- oder Metallions, bevorzugt für Natrium, Kalium, ½ Magnesium, ½ Calcium, ½ Zink, oder für ein Ammoniumion ($NH_4^+$).

Es ist von Vorteil, die Verbindungen der Formeln (I) und (II) in bestimmten Mengenbereichen zueinander

einzusetzen. Bevorzugt ist in diesem Zusammenhang ein molares Mischungsverhältnis von 20:1 bis 1:20, weiter bevorzugt von 10:1 bis 1:10 und ganz besonders bevorzugt von 3:1 bis 1:3 . Das molare Verhältnis aus allen im Mittel enthaltenen Verbindungen der Formel (I) zu allen im Mittel enthaltenen Verbindungen der Formel (II), d.h. das molare Verhältnis (I)/(II), liegt daher bevorzugt bei einem Wert von 20:1 bis 1:20, weiter bevorzugt bei einem Wert von 10:1 bis 1:10 und ganz besonders bevorzugt von 3:1 bis 1:3.
In einer weiteren Ausführungsform ist ein erfindungsgemäßes Mittel daher dadurch gekennzeichnet, dass das molare Verhältnis aus allen im Mittel enthaltenen Verbindungen der Formel (I) zu allen im Mittel enthaltenen Verbindungen der Formel (II), d.h. das molare Verhältnis (I)/(II), bei einem Wert von 20:1 bis 1:20, weiter bevorzugt bei einem Wert von 10:1 bis 1:10 und ganz besonders bevorzugt von 3:1 bis 1:3 liegt.

Mit bestimmten Verbindungen der Formel (I) lässt sich ein ganz besonders gutes Entfärbeergebnis von oxidativ gefärbten Haaren erzielen. Ganz besonders bevorzugt ist daher der Einsatz einer oder mehrere Verbindungen aus der Gruppe aus

- $HN(CH_2SO_2Na)_2$, Dinatrium [(sulfinatomethyl)amino]methansulfinat
- $HN(CH_2SO_2K)_2$, Dikalium [(sulfinatomethyl)amino]methansulfinat
- $HN(CH_2SO_2H)_2$, [(Sulfinomethyl)amino]methansulfinsäure
- $N(CH_2SO_2Na)_3$, Trinatrium [bis(sulfinatomethyl)amino]methansulfinat
- $N(CH_2SO_2K)_3$, Trikalium [bis(sulfinatomethyl)amino]methansulfinat
- $N(CH_2SO_2H)_3$, [Bis(sulfinomethyl)amino]methansulfinsäure
- $HN(CH(CH_3)SO_2Na)_2$, Dinatrium 1-[(1-sulfinatoethyl)amino]ethan-1-sulfinat
- $HN(CH(CH_3)SO_2K)_2$, Dikalium 1-[(1-sulfinatoethyl)amino]ethan-1-sulfinat
- $HN(CH(CH_3)SO_2H)_2$, 1-[(1-Sulfinoethyl)amino]ethan-1-sulfinsäure
- $N(CH(CH_3)SO_2Na)_3$, Trinatrium 1-[Bis(1-sulfinatoethyl)amino]ethan-1-sulfinat und/oder
- $N(CH(CH_3)SO_2K)_3$, Trikalium 1-[Bis(1-sulfinatoethyl)amino]ethan-1-sulinat
- $N(CH(CH_3)SO_2H)_3$, 1-[Bis(1-sulfinoethyl)amino]ethan-1-sulfinsäure

In einer besonders bevorzugten Ausführungsform ist ein erfindungsgemäßes Verfahren dadurch gekennzeichnet, dass das Entfärbemittel (a) mindestens eine Verbindung der Formel (I) enthält, die ausgewählt ist aus der Gruppe aus

- $HN(CH_2SO_2Na)_2$, Dinatrium [(sulfinatomethyl)amino]methansulfinat
- $HN(CH_2SO_2K)_2$, Dikalium [(sulfinatomethyl)amino]methansulfinat
- $HN(CH_2SO_2H)_2$, [(Sulfinomethyl)amino]methansulfinsäure
- $N(CH_2SO_2Na)_3$, Trinatrium [bis(sulfinatomethyl)amino]methansulfinat
- $N(CH_2SO_2K)_3$, Trikalium [bis(sulfinatomethyl)amino]methansulfinat
- $N(CH_2SO_2H)_3$, [Bis(sulfinomethyl)amino]methansulfinsäure
- $HN(CH(CH_3)SO_2Na)_2$, Dinatrium 1-[(1-sulfinatoethyl)amino]ethan-1-sulfinat
- $HN(CH(CH_3)SO_2K)_2$, Dikalium 1-[(1-sulfinatoethyl)amino]ethan-1-sulfinat
- $HN(CH(CH_3)SO_2H)_2$, 1-[(1-Sulfinoethyl)amino]ethan-1-sulfinsäure
- $N(CH(CH_3)SO_2Na)_3$, Trinatrium 1-[Bis(1-sulfinatoethyl)amino]ethan-1-sulfinat und/oder
- $N(CH(CH_3)SO_2K)_3$, Trikalium 1-[Bis(1-sulfinatoethyl)amino]ethan-1-sulinat und/oder
- $N(CH(CH_3)SO_2H)_3$, 1-[Bis(1-sulfinoethyl)amino]ethan-1-sulfinsäure.

Auch die im erfindungsgemäßen Mittel enthaltenen Verbindungen der Formel (II) werden bevorzugt aus einer bestimmten Gruppe von Verbindungen ausgewählt. Ganz besonders bevorzugt ist daher der Einsatz einer oder mehrere Verbindungen aus der Gruppe aus

- $HN(CH_2SO_3Na)_2$, Dinatrium [(sulfonatomethyl)amino]methansulfonat
- $HN(CH_2SO_3K)_2$, Dikalium [(sulfonatomethyl)amino]methansulfonat
- $HN(CH_2SO_3H)_2$, [(Sulfomethyl)amino]methansulfonsäure
- $N(CH_2SO_3Na)_3$, Trinatrium [bis(sulfonatomethyl)amino]methansulfonat
- $N(CH_2SO_3K)_3$, Trikalium [bis(sulfonatomethyl)amino]methansulfonat
- $N(CH_2SO_3H)_3$, [Bis(sulfomethyl)amino]methansulfonsäure
- $HN(CH(CH_3)SO_3Na)_2$, Dinatrium 1-[(1-sulfonatoethyl)amino]ethan-1-sulfonat
- $HN(CH(CH_3)SO_3K)_2$, Dikalium 1-[(1-sulfonatoethyl)amino]ethan-1-sulfonat
- $HN(CH(CH_3)SO_3H)_2$, 1-[(1-Sulfoethyl)amino]ethan-1-sulfonsäure
- $N(CH(CH_3)SO_3Na)_3$, Trinatrium 1-[bis(1-sulfonatoethyl)amino]ethan-1-sulfonat
- $N(CH(CH_3)SO_3K)_3$, Trikalium 1-[bis(1-sulfonatoethyl)amino]ethan-1-sulfonat
- $N(CH(CH_3)SO_3H)_3$, 1-[Bis(1-sulfoethyl)amino]ethan-1-sulfonsäure

In einer besonders bevorzugten Ausführungsform ist ein erfindungsgemäßes Verfahren dadurch gekennzeichnet, dass das Entfärbemittel (b) mindestens eine Verbindung der Formel (II) enthält, die ausgewählt ist aus der Gruppe aus

- $HN(CH_2SO_3Na)_2$, Dinatrium [(sulfonatomethyl)amino]methansulfonat
- $HN(CH_2SO_3K)_2$, Dikalium [(sulfonatomethyl)amino]methansulfonat
- $HN(CH_2SO_3H)_2$, [(Sulfomethyl)amino]methansulfonsäure
- $N(CH_2SO_3Na)_3$, Trinatrium [bis(sulfonatomethyl)amino]methansulfonat
- $N(CH_2SO_3K)_3$, Trikalium [bis(sulfonatomethyl)amino]methansulfonat
- $N(CH_2SO_3H)_3$, [Bis(sulfomethyl)amino]methansulfonsäure
- $HN(CH(CH_3)SO_3Na)_2$, Dinatrium 1-[(1-sulfonatoethyl)amino]ethan-1-sulfonat
- $HN(CH(CH_3)SO_3K)_2$, Dikalium 1-[(1-sulfonatoethyl)amino]ethan-1-sulfonat
- $HN(CH(CH_3)SO_3H)_2$, 1-[(1-Sulfoethyl)amino]ethan-1-sulfonsäure.
- $N(CH(CH_3)SO_3Na)_3$, Trinatrium 1-[bis(1-sulfonatoethyl)amino]ethan-1-sulfonat
- $N(CH(CH_3)SO_3K)_3$, Trikalium 1-[bis(1-sulfonatoethyl)amino]ethan-1-sulfonat und/oder
- $N(CH(CH_3)SO_3H)_3$, 1-[Bis(1-sulfoethyl)amino]ethan-1-sulfonsäure

Das bzw. die Reduktionsmittel der Formel (I) werden bevorzugt in bestimmten Mengenbereichen eingesetzt. Ein entfärbender Effekt ist bereits bei kleinen Einsatzmengen zu beobachten. Um eine ausreichende und starke Entfärbewirkung zu erhalten, ist es jedoch von Vorteil, wenn das Entfärbemittel ein oder mehrere Reduktionsmittel der Formel (I) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-% enthält. Berechnungsgrundlage der Mengenangaben in Gew.-% ist hierbei das Gesamtgewicht aller im Mittel enthaltenden Reduktionsmittel der Formel (I), das zum Gesamtgewicht des Mittels in Relation gesetzt wird.

In einer weiteren besonders bevorzugten Ausführungsform ist ein erfindungsgemäßes Verfahren dadurch gekennzeichnet, dass das Entfärbemittel - bezogen auf das Gesamtgewicht des Mittels - eine oder mehrere Verbindungen der Formel (I) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-% enthält.

Auch die im Mittel enthaltenen Verbindungen der Formel (II) werden bevorzugt in bestimmten Mengenbereichen eingesetzt. Es ist bevorzugt, wenn das Entfärbemittel die Verbindung(en) der Formel (II) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-%. Berechnungsgrundlage der Mengenangaben in Gew.-% ist hierbei das Gesamtgewicht aller im Mittel enthaltenden Verbindungen der Formel (II), das zum Gesamtgewicht des Mittels in Relation gesetzt wird.

In einer weiteren bevorzugten Ausführungsform ist ein erfindungsgemäßes Verfahren dadurch gekennzeichnet, dass das Entfärbemittel - bezogen auf das Gesamtgewicht des Mittels - eine oder mehrere Verbindungen der Formel (II) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-% enthält.

Bei einem ganz besonders bevorzugten Reduktionsmittel, das Verbindungen der Formeln (I) und (II) umfasst, handelt es sich um den Rohstoff Cyclanon Clear ECO, der alternativ auch als Cyclanon ECO bezeichnet wird und kommerziell von der Firma BASF erhältlich ist. Cyclanon ECO ist ein Gemisch der Verbindungen $N(CH_2SO_2Na)_3$ und $N(CH_2SO_3Na)_3$ im molaren Verhältnis von 1:1. Zusätzlich kann das erfindungsgemäße Mittel auch einen oder mehrere weitere Reduktionsmittel (c) aus der Gruppe aus Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Natriumdisulfit, Kaliumdisulfit, Ammoniumdisulfit, Hydroxymethansulfinsäure, Aminomethansulfinsäure, Cystein, Thiomilchsäure, Thioglycolsäure (Alternativname: Sulfanylessigsäure), Oxalsäure, Oxalessigsäure und/oder Ascorbinsäure enthalten.

Bei Natriumdithionit handelt es sich um ein anorganischen Reduktionsmittel mit der Summenformel $Na_2S_2O_4$ und der CAS-Nr. 7775-14-6.

Bei Zinkdithionit handelt es sich um ein anorganisches Reduktionsmittel mit der Summenformel $ZnS_2O_4$ und der CAS-Nr. 7779-86-4.

Bei Kaliumdithionit handelt es sich um ein anorganisches Reduktionsmittel mit der Summenformel $K_2S_2O_4$ und der CAS-Nr. 14293-73-3.

Bei Natriumsulfit handelt es sich um ein anorganisches Reduktionsmittel mit der Summenformel $Na_2SO_3$ und der CAS-Nr. 7757-83-7.

Bei Natriumhydrogensulfit handelt es sich um ein anorganisches Reduktionsmittel mit der Summenformel $NaHSO_3$ und der CAS-Nr. 7631-90-5. Natriumhydrogensulfit wird bevorzugt in Form einer wässrigen Lösung eingesetzt.

Bei Kaliumsulfit handelt es sich um ein ein anorganisches Reduktionsmittel mit der Summenformel $K_2SO_3$ und der CAS-Nr. 10117-38-1.

Bei Kaliumhydrogensulfit handelt es sich um ein anorganisches Reduktionsmittel mit der Summenformel $KHSO_3$

und der CAS-Nr. 7773-03-7.

Bei Ammoniumsulfit handelt es sich um ein anorganisches Reduktionsmittel mit der Summenformel $(NH_4)_2SO_3$ und der CAS-Nr. 10196-04-0.

Bei Natriumthiosulfat handelt es sich um ein anorganisches Reduktionsmittel mit der Summenformel $Na_2S_2O_3$ und der CAS-Nr. 7772-98-7.

Bei Kaliumthiosulfat handelt es sich um ein anorganisches Reduktionsmittel mit der Summenformel $K_2S_2O_3$ und der CAS-Nr. 10294-66-3.

Bei Ammoniumthiosulfat handelt es sich um ein anorganisches Reduktionsmittel mit der Summenformel $(NH_4)_2S_2O_3$ und der CAS-Nr. 7783-18-8.

Bei Hydroxymethansulfinsäure handelt es sich um ein organisches Reduktionsmittel mit der Formel HO-$CH_2$-S(O)OH und der CAS-Nr. 79-25-4. Alternativ wird Hydroxymethansulfinsäure auch als Formaldehydsulfoxylsäure bezeichnet. Erfindungsgemäß ist sowohl der Einsatz der Hydroxymethansulfinsäure selbst als auch der Einsatz der physiologisch verträglichen Salze von Hydroxymethansulfinsäure, beispielsweise des Natriumsalzes und/oder des Zinksalzes. Der Einsatz von Natriumformaldehydsulfoxylat (Natrium Hydroxymethansulfinat, dem Natriumsalz der Hydroxymethansulfinsäure) und/oder Zinkformeladehydsulfoxylat (Zink Hydroxymethansulfinat, dem Zinksalz der Hydroxymethansulfinsäure) ist demnach ebenfalls erfindungsgemäß.

Bei Aminomethansulfinsäure handelt es sich um ein organisches Reduktionsmittel mit der Formel $H_2N$-$CH_2$-S(O)OH und der CAS-Nr. 118201-33-5. Erfindungsgemäß ist sowohl der Einsatz der Aminomethansulfinsäure selbst als auch der Einsatz der physiologisch verträglichen Salze von Aminomethansulfinsäure, beispielsweise des Natriumsalzes und/oder des Zinksalzes. Der Einsatz von Natrium Aminomethansulfinat (dem Natriumsalz der Aminomethansulfinsäure) und/oder Zink Aminomethansulfinat (dem Zinksalz der Aminomethansulfinsäure) ist deshalb ebenfalls erfindungsgemäß.

Unter Cystein (2-Amino-3-sulfanylpropionsäure) wird erfindungsgemäß D-Cystein, L-Cystein und/oder ein Gemisch aus D- und L-Cystein verstanden.

Unter Thiomilchsäure (2-Sulfanylpropionsäure) wird D-Thio-Milchsäure, L-Thio-Milchsäure und/oder ein Gemisch aus D- und L-Thiomilchsäure verstanden. Erfindungsgemäß ist sowohl der Einsatz der Thiomilchsäure selbst als auch der Einsatz von Thiomilchsäure in Form eines physiologisch verträglichen Salzes hiervon. Ein bevorzugtes Salz der Thiomilchsäure ist Ammoniumthiolactat. Bei Ammoniumthiolactat handelt es sich um das Ammoniumsalz der Thiomilchsäure (d.h. das Ammoniumsalz der 2-Sulfanylpropionsäure) (Formel XX).

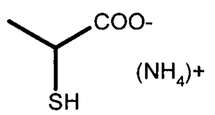

(Formel XX)

Von der Definition Ammoniumthiolactat mit umfasst sind sowohl die Ammoniumsalze der D-Thiomilchsäure als auch die Ammoniumsalze der L-Thiomilchsäure sowie deren Gemische.

Unter Thioglycolsäure (Sulfanylessigsäure, 2-Mercapto-essigäure) wird ein organisches Reduktionsmittel der Formel HS-$CH_2$-COOH verstanden, die Verbindung besitzt die CAS-Nr. 68-11-1. Auch bei Thioglycolsäure ist sowohl der Einsatz von Thioglycolsäure selbst auch der Einsatz eines physiologisch verträglichen Salzes der Thioglycolsäure erfindungsgemäß. Als physiologisch verträgliche Salze der Thiolgycolsäure können beispielsweis Natriumthioglycolat, Kaliumthioglycolat und/oder Ammoniumthioglycolat eingesetzt werden. Ammoniumthioglylat ist ein bevorzugtes physiologisch verträgliches Salze der Thioglycolsäure.

Bei Ammoniumthioglycolat handelt es sich um das Ammoniumsalz der Thiglycolsäure (d.h. das Ammoniumsalz der Sulfanylessigsäure) (Formel XXX).

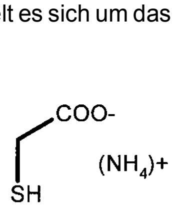

(Formel XXX)

Unter Oxalsäure wird die reduzierend wirkende Säure HOOC-COOH verstanden. Oxalsäure wird alternativ auch als Ethandisäure bezeichnet und trägt die CAS-Nr. 144-62-7.

Alternativnamen für Oxalessigsäure sind auch Oxobutandisäure oder Oxobernsteinsäure, diese Säure wirkt ebenfalls reduzierend und trägt die CAS-Nr. 328-42-7.

Unter Ascorbinsäure wird erfindungsgemäß insbesondere (R)-5-[(S)-1,2-Dihydroxyethyl]-3,4-dihydroxy-5*H*-furan-2-on (weitere Alternativnamen: Vitamin C, L-Ascorbinsäure) mit der CAS-Nr. 50-81-7 verstanden.

In einer weiteren bevorzugten Ausführungsform ist ein erfindungsgemäßes Verfahren daher dadurch gekennzeichnet, dass das Entfärbemittel zusätzlich ein oder mehrere weitere Reduktionsmittel (c) aus der Gruppe aus Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Natriumdisulfit, Kaliumdisulfit, Ammoniumdisulfit, Hydroxymethansulfinsäure, Aminomethansulfinsäure, Cystein, Thiomilchsäure, Thioglycolsäure (Alternativname: Sulfanylessigsäure) und/oder Ascorbinsäure enthält.

Das oder die weiteren Reduktionsmittel aus der vorgenannten Gruppe (c) können in einer Gesamtmenge von 0,1 bis 20,0 Gew.-%, bevorzugt von 0,5 bis 10,0 Gew.-% und besonders bevorzugt von 1,0 bis 8,0 Gew.-% - jeweils bezogen auf das Gesamtgewicht des Mittels - im Mittel enthalten sein.

Die erfindungsgemäßen Mittel enthalten weiterhin bevorzugt mindestens ein Tensid aus der Gruppe der anionischen, kationischen, amphoteren und/oder zwitterionischen Tenside.

In einer weiteren besonders bevorzugten Ausführungsform ist ein erfindungsgemäßes Verfahren dadurch gekennzeichnet, dass das Entfärbemittel zusätzlich mindestens ein Tensid aus der Gruppe der anionischen, amphoteren und/oder zwitterionischen, nichtionischen und/oder kationischen Tenside enthält.

Tenside sind amphiphile (bifunktionelle) Verbindungen, die aus mindestens einem hydrophoben und mindestens einem hydrophilen Molekülteil bestehen. Bei dem hydrophoben Rest handelt es sich bevorzugt eine Kohlenwasserstoffkette mit 8-24 Kohlenstoff-Atomen, die gesättigt oder ungesättigt, linear oder verzweigt sein kann. Besonders bevorzugt ist diese $C_8$-$C_{24}$-Alkylkette linear.

Bei anionischen Tensiden umfasst der hydrophile Molekülteil eine negativ geladene hydrophile Kopfgruppe. Bei der negativ geladenen hydrophilen Kopfgruppe kann es sich beispielsweise um eine Carbonsäuregruppe bzw. das Salz einer Carbonsäuregruppe, eine Sulfonsäuregruppe bzw. das Salz der Sulfonsäuregruppe, eine Schwefelsäureestergruppierung bzw. das Salz hiervon, eine Phosphonsäuregruppe bzw. das Salz der Phosphonsäuregruppe, oder eine Phosphorsäureestergruppierung bzw. das Salz hiervon handeln.

Üblicherweise umfasst das erfindungsgemäße kosmetische Mittel einen wässrigen Träger. In wässriger Lösung liegen die vorgenannten hydrophilen Kopfgruppen des anionischen Tensids - wie beispielsweise die Carbonsäure und die Salze der Carbonsäuren - in einem Gleichgewicht vor, dessen Lage vom pH-Wert des Mittels mitbestimmt wird. Wird als anionisches Tensid somit beispielsweise eine Fettsäure eingesetzt, so liegt ein geringer Teil der Fettsäure in wässriger Lösung in Form der protonierten Fettsäure vor, wohingegen der Großteil der Fettsäure in wässriger Lösung deprotoniert und auf diesem Wege in das Salz der Fettsäure überführt wird. Aus diesem Grund umfasst die Definition eines anionischen Tensid auch ein Tenside mit einer - noch protonierten - Säuregruppe.

Ein anionisches Tensid (b) im Sinne der vorliegenden Erfindung enthält keine kationischen Gruppierungen, d.h. zwitterionische Tenside sind von der Definition eines anionischen Tensids nicht mit umfasst.

Erfindungsgemäße anionische Tenside sind demnach gekennzeichnet durch die Anwesenheit einer wasserlöslich machenden, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder

Polyglykolether-Gruppen, Ester-, Ether- und Amidgruppen sowie Hydroxylgruppen enthalten sein. Typische Beispiele für anionische Tenside sind Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Beispiele für erfindungsgemäße anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel R-O-$(CH_2$-$CH_2O)_x$-$CH_2$-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche $C_{12}$ - $C_{18}$-Fettsäurefraktion einsetzt, erhält man die erfindungsgemäß bevorzugt geeigneten $C_{12}$ - $C_{18}$-Acylisethionate,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen. Sulfobernsteinsäuremono- und -dialkylester können durch Umsetzung von Maleinsäureanhydrid mit einem Fettalkohol mit 8 - 24 C-Atomen zum Maleinsäuremonoester des Fettalkohols und Weiterreaktion mit Natriumsulfit zum Sulfobernsteinsäureester hergestellt werden. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind,

- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R\text{-}O(CH_2\text{-}CH_2O)_x\text{-}OSO_3H$, in der R eine bevorzugt lineare Alkylgruppe mit 8 bis 30 C-Atomen und x = 0 oder 1 bis 12 ist,
- Hydroxysulfonate im wesentlichen entsprechend mindestens einer der beiden folgenden Formeln oder deren Mischungen sowie deren salzen, $CH_3\text{-}(CH_2)_y\text{-}CHOH\text{-}(CH_2)_p\text{-}(CH\text{-}SO_3M)\text{-}(CH_2)_z\text{-}CH_2\text{-}O\text{-}(C_nH_{2n}O)_x\text{-}H$, und/oder $CH_3\text{-}(CH_2)_y\text{-}(CH\text{-}SO_3M)\text{-}(CH_2)_p\text{-}CHOH\text{-}(CH_2)_z\text{-}\ CH_2\text{-}O\text{-}(C_nH_{2n}O)_x\text{-}H$ wobei in beiden Formeln y und z = 0 oder ganze Zahlen von 1 bis 18, p = 0, 1 oder 2 und die Summe (y+z+p) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C1 bis C4 Alkyl-, Alkenyl- oder Arylresten,
- sulfatierte Hydroxyalkylpolyethylen- und/oder Hydroxyalkylenpropylenglykolether der Formel $R^1\text{-}(CHOSO_3M)\text{-}CHR^3\text{-}(OCHR^4\text{-}CH_2)n\text{-}OR^2$ mit $R^1$, einem linearen Alkylrest mit 1 bis 24 C-Atomen, $R^2$ für einen linearen oder verzweigten, gesättigten Alkylrest mit 1 bis 24 C-Atomen, $R^3$ für Wasserstoff oder einen linearen Alkylrest mit 1 bis 24 C-Atomen, $R^4$ für Wasserstoff oder einen Methylrest und M für Wasserstoff, Ammonium, Alkylammonium, Alkanolammonium, worin die Alkyl- und Alkanolreste je 1 bis 4 C-Atome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in $R^1$ und $R^3$ enthaltenen C-Atome 2 bis 44 beträgt,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

$$R^1(OCH_2CH_2)_n\text{-}O\text{-}(PO\text{-}OX)\text{-}OR^2,$$

  in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^2$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen $C_1$ bis $C_4$ - Kohlenwasserstoffrest, steht,
- sulfatierte Fettsäurealkylenglykolester der Formel $RCO(AlkO)_nSO_3M$
  in der RCO- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie $+NR^3R^4R^5R^6$, mit $R^3$ bis $R^6$ unabhängig voneinander stehend für Wasserstoff oder einen C1 bis C4 - Kohlenwasserstoffrest,
- Monoglyceridsulfate und Monoglyceridethersulfate der Formel

$$R^8OC\text{-}(OCH_2CH_2)_x\text{-}OCH_2\text{-}[CHO(CH_2CH_2O)_yH]\text{-}CH_2O(CH_2CH_2O)_z\text{-}SO_3X,$$

  in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der $R^8CO$ für einen linearen Acylrest mit 8 bis 18 Kohlenstoffatomen steht,
  Amidethercarbonsäuren, $R^1\text{-}CO\text{-}NR^2\text{-}CH_2CH_2\text{-}O\text{-}(CH_2CH_2O)_nCH_2COOM$, mit $R^1$ als geradkettigen oder verzweigten Alkyl- oder Alkenylrest mit einer Zahl an Kohlenstoffatomen in der Kette von 2 bis 30, n steht für eine ganze Zahl von 1 bis 20 und $R^2$ steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie $+NR^3R^4R^5R^6$, mit $R^3$ bis $R^6$

unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo®erhältlich, und

- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

Das erfindungsgemäße Mittel kann auch ein oder mehrere amphotere und/oder zwitterionische Tenside enthalten. Im Fall der zwitterionischen Tenside umfasst der hydrophile Molekülteil eine zwitterionische Struktureinheit, d.h. eine Struktureinheit, die sowohl einen kationisch geladenen als auch einen anionisch geladenen Molekülteil umfasst. Erfindungsgemäß besonders geeignete zwitterionische Tenside (b) sind dadurch gekennzeichnet, dass sie einen kationisch geladenen Molekülteil in Form einer quartären Ammoniumgruppe besitzen und ihr anionischer Molekülteil in Form einer Gruppierung $-SO_3^-$ oder $-COO^-$ vorliegt.

Eine Ammoniumgruppe ist quartär, wenn eine Gruppierung des Typs $(R_aR_bR_cR_dN)^+$ vorliegt, d.h. wenn alle vier H-Atome des $NH_4$-Ions, von dem die quartäre Ammoniumgruppe abgeleitet ist, durch organische Reste R (bzw. $R_a$ bis $R_d$) ersetzt wird.

Die Gruppierung $-SO3^-$ des zwitterionischen Tensids kann direkt an ein Kohlenstoffatom gebunden vorliegen. In diesem Fall handelt es sich bei dem anionischen Teil der zwitterionischen Verbindung um eine deprotonierte Sulfonsäuregruppierung.

Besonders geeignete zwitterionische Tenside sind beispielsweise Betaine, N-Alkyl-N,N-dimethylammoniumglycinate, N-Acyl-aminopropyl-N,N-dimethylammoniumglycinate, und 2-Alkyl-3-carboxymethyl-3-hydroxyethyl-imidazoline.

Erfindungsgemäß geeignete Verfahren sind weiterhin dadurch gekennzeichnet, dass das Entfärbemittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und $C_{12}$-$C_{18}$-Acylsarcosin.

Weiterhin hat es sich als vorteilhaft erwiesen, wenn das Mittel weitere, nichtionogene grenzflächenaktive Stoffe, enthält. Bevorzugte nichtionische Tenside sind Alkylpolyglykoside sowie Alkylenoxid-Anlagerungsprodukte an Fettalkohole, Fettsäuren und Fettsäureglyceride mit jeweils 2 bis 50 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Ganz besonders bevorzugt ist es, wenn das Mittel als nichtionisches Tensid ein ethoxyliertes Rizinusöl mit jeweils 2 bis 50 Mol Ethylenoxyid pro Mol Fettsäure oder ein ethoxyliertes, hydriertes Rizinusöl mit jeweils 2 bis 50 Mol Ethylenoxyid pro Mol Fettsäure enthält. Der Einsatz von PEG-40 Castor Oil ist in diesem Zusammenhang besonders bevorzugt.

Die erfindungsgemäßen Mittel können auch ein oder mehrere kationische Tenside enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren an Grenzflächen und aggregieren in wässriger Lösung oberhalb der kritischen Micellbildungskonzentration zu positiv geladenen Micellen.

Beispiele für Kationtenside sind

- quartäre Ammoniumverbindungen, die als hydrophobe Reste ein oder zwei Alkylketten mit einer Kettenlänge von 8 bis 28 C-Atomen tragen können
- quartäre Phosphoniumsalze, substituert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das Kationtensid auch weitere ungeladene funktionelle Gruppen beinhalten, dies ist beispielsweise bei Esterquats der Fall. Erfindungsgemäß werden als kationische Tenside insbesondere solche vom Typ der quartären Ammoniumverbindungen, der Esterquats und der Amidoamine eingesetzt. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar. Weitere erfindungsgemäß verwendbare kationische Tenside stellen die quaternisierten Proteinhydrolysate dar.

Die anionischen, amphoteren bzw. zwitterionischen, nichtionischen und kationischen Tenside können jeweils in einer Menge von 0,1 bis 15,0 Gew.-%, bevorzugt von 0,25 bis 12,0 Gew.-%, weiter bevorzugt von 1,25 bis 10,0 Gew.-% und besonders bevorzugt von 1,50 bis 9,0 Gew.-% - jeweils bezogen auf das Gesamtgewicht des Mittels - enthalten sein.

Weiterhin hat sich herausgestellt, dass der Einsatz von Polyolen die Entfärbewirkung weiter unterstützt. Aus diesem Grund ist es bevorzugt, wenn die erfindungsgemäßen Entfärbemittel zusätzlich ein oder mehrere Polyole enthalten.

Unter einem Polyol wird eine Verbindung mit mindestens zwei aliphatischen (d.h. nicht phenolischen) OH-Gruppen verstanden.

Beispiele für geeignete erfindungsgemäße Polyole sind insbesondere Ethylengylcöl, 1,2-Propylenglycol, 1,3-Propandiol, 1,2-Butandiol, 1,3-Butandiol, 1,2-Pentandiol, 1,3-Pentandiol, 1,4-Pentandiol, 1,5-Pentandiol, 1,2-Hexandiol, 1,3-hexandiol, 1,4-Hexandiol, 1,5-Hexandiol und 1,6-Hexandiol. Geeignet sind aber auch Polyethylenglycol und Polypropylenglycol.

In einer weiteren Ausführungsform ist ein erfindungsgemäßes Entfärbemittel daher dadurch gekennzeichnet, dass es zusätzlich ein oder mehrere Polyole aus der Gruppe Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol und/oder Polypropylenglycol enthält. Die Polyole werden in den erfindungsgemäßen Mitteln bevorzugt in einer Gesamtmenge von 0,5 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des Mittels - eingesetzt.

Abhängig von den zuvor für die Färbung verwendeten Farbstoffen und abhängig vom Zustand, dem Schädigungsgrad und der Dicke der Haare kann der Entfärbeprozess bei unterschiedlichen Personen unterschiedlich lange dauern. Auch wenn der Entfärbeprozess generell innerhalb der üblichen Anwendungszeiten von bis zu 90 Minuten abgeschlossen sein sollte, ist es für den Anwender des Entfärbemittels doch um ein vielfaches komfortabler, den Entfärbevorgang direkt zu beobachten und das Entfärbemittel direkt nach Abschluss der Entfärbung - gegebenenfalls bereits nach 20 oder 30 Minuten - abspülen zu können.

Die direkte Beobachtung des Entfärbeprozesses wird für den Anwender oder Friseur möglich, wenn das Entfärbemittel in Form eines transparenten Gels konfektioniert wird.

In einer weiteren bevorzugten Ausführungsform ist ein erfindungsgemäßes Entfärbemittel daher dadurch gekennzeichnet, dass es sich um eine klare, fließfähige Gelformulierung mit einem Transmissionsgrad T von mindestens 70 %, bevorzugt von mindestens 75 %, weiter bevorzugt von mindestens 80 % und besonders bevorzugt mindestens 85 % handelt, wobei sich der Transmissionsgrad T über die folgende Formel berechnet

$$T = \Phi ex/\Phi in$$

mit

$\Phi ex$ gleich der Strahlungsintensität des aus dem Mittel austretenden, durchgelassenen Lichtstrahls und

$\Phi in$ gleich der Strahlungsintensität des in das Mittel einfallenden Lichtstrahls.

Unter Gelen werden Systeme verstanden, die aus einem festen, kolloidisch verteilten Stoff, bei dem es sich um ein Verdickungsmittel bzw. einem Geliermittel handelt, und einer Flüssigkeit (Wasser oder Wasser-Lösungsmittel-Gemische) verstanden. Hierbei bildet das Verdickungs- bzw. Geliermittel in der Flüssigkeit ein räumliches Netzwerk.

Die Gele im Sinne der vorliegenden Erfindung sind fließfähig, was bedeutet, dass sie bevorzugt eine Viskosität von 100 mPas bis 15.000 mPas, besonders bevorzugt von 3.000 mPas bis 8.000 mPas (bei Messungen mit einem Rotationsviskosimeter von Brookfield, Spindelgröße 4, bei 25°C und 20 Upm) besitzen.

Die Transmission beschreibt die Durchlässigkeit des Gels für das Messlicht (bevorzugt Tageslicht), und wird als Transmissionsgrad T ausgedrückt. Der Transmissionsgrad stellt einen Verhältniswert $T = \Phi ex/\Phi in$ dar, wobei die

Strahlungsintensität des aus dem Mittel austretenden, durchgelassenen Lichtstrahls (Φex) zur Strahlungsintensität des in das Mittel einfallenden Lichtstrahls (Φin) ins Verhältnis gesetzt wird. Die Messung erfolgt mit Tageslicht (Tageslichtlampe) bei einer Schichtdicke von 1 cm (d.h. das zu vermessende Gel wird in eine Küvette eingefüllt, so dass das Gel in einer Schichtdicke von 1 cm vorliegt und wird dann mit einem handelsüblichen Photometer vermessen). Bei einem Transmissionsgrad von mindestens 70%, bevorzugt von mindestens 75 %, weiter bevorzugt von mindestens 80 % und besonders bevorzugt mindestens 85 % ist das Gel so transparent, dass der Konsument den Entfärbeprozess des Haares direkt durch das transparente, auf das Haar aufgetragene Gel hindurch beobachten kann. Auf diese Weise kann er die Beendigung des Entfärbeprozesses direkt optisch wahrnehmen, ohne des Entfärbemittel von einer Probesträhne des behandelten Haares abspülen zu müssen.

Als Geliermittel bzw. Verdickungsmittel zur Herstellung des transparenten Gels können beispielsweise anionische, synthetische Polymere, der kationische, synthetische Polymere, der nichtionische Guargums, Skleroglucangums, Xanthangums, Gummi arabicum, Carrageen-Gummi, Agar-Agar, Johannisbrotkernmehl, der Pektine, der Alginate, der Stärke, der Cellulosen und Cellulosederivate und/oder nichtionische, synthetische Polymere eingesetzt werden.

In einer weiteren bevorzugten Ausführungsform ist ein erfindungsgemäßes Verfahren daher dadurch gekennzeichnet, dass das Entfärbemittel zusätzlich mindestens ein Verdickungsmittel enthält, das ausgewählt ist aus der Gruppe der anionischen, synthetischen Polymere, der kationischen, synthetischen Polymere, der nichtionischen Guargums, der Skleroglucangums, der Xanthangums, Gummi arabicum, Carrageen-Gummi, Agar-Agar, Johannisbrotkernmehl, der Pektine, der Alginate, der Stärke, der Cellulosen und Cellulosederivate und/oder der nichtionischen, synthetischen Polymere.

Bei den erfindungsgemäßen Mitteln handelt es sich um Entfärbemittel, die zur Entfärbung von zuvor gefärbten keratinischen Fasern, insbesondere menschlichen Haaren eingesetzt werden. Bei den gefärbten Keratinfasern handelt es sich üblicherweise um Fasern, die zuvor mit herkömmlichen, dem Fachmann bekannten Oxidationsfarbstoffen und/oder direktziehenden Farbstoffen gefärbt wurden. Die Entfärbemittel sind geeignet zur Entfernung von Färbungen, die mit Oxidationsfarbstoffen auf Basis von Entwickler- und Kupplerkomonenten auf den Keratinfasern erzeugt wurden. Wenn als Entwickler die folgenden Verbindungen einsetzt wurden, lassen sich die hiermit erzeugten Färbungen durch Einsatz des Entfärbemittel gut, effektiv und nahezu ohne spätere Nachdunklung entfernen: p-Phenylendiamin, p-ToluylendiaminN,N-Bis-(β-hydroxyethyl)-p-phenylendiamin, 4-N,N-Bis-(β-hydroxyethyl)-amino-2-methylanilin, 2-(β-Hydroxyethyl)-p-phenylendiamin, 2-(a,β-Dihydroxyethyl)-p-phenylendiamin, 2-Hydroxymethyl-p-phenylendiamin, Bis-(2-hydroxy-5-aminophenyl)-methan, p-Aminophenol, 4-Amino-3-methylphenol, 2,4,5,6-Tetraaminopyrimidin, 4-Hydroxy-2,5,6-triaminopyrimidin, 2-Hydroxy-4,5,6-triaminopyrimidin und/oder 4,5-Diamino-1-(β-hydroxyethyl)-pyrazol.

Wenn als Kuppler die folgenden Verbindungen einsetzt wurden, lassen sich die hiermit erzeugten Färbungen ebenfalls mit sehr gutem Entfärbeergebnis entfernen: m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol. 1-Naphthol, 1,5-Dihydroxynaphthalin, 2,7-Dihydroxynaphthalin, 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgmäßen Entfärbemittel sind zur Entfernung dieser Färbungen gedacht und enhalten selbst daher bevorzugt keine Farbstoffe, d.h. keine Oxidationsfarbstoffvorprodukte vom Entwicklertyp und vom Kupplertyp und auch keine direktziehenden Farbstoffe.

In einer weiteren bevorzugten Ausführungsform ist ein erfindungsgemäßes Entfärbemittel daher dadurch gekennzeichnet, dass die Gesamtmenge aller im Mittel enthaltenen direktziehenden Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels - liegt.

Alle vorstehend beschriebenen Ausführungsformen und Mengenangaben sind bezogen auf das anwendungsbereite Entfärbemittel, d.h. das Mittel, das zur Anwendung bereit ist und direkt vom Anwender auf die zuvor gefärbten keratinischen Fasern appliziert werden kann. Die Entfärbewirkung dieser anwendungsbereiten Entfärbemittel ist eine Funktion des pH-Wertes, die im sauren Milieu ihr Optimum durchläuft. Die pH-Werte, bei welchen die stärkste Entfärbung beobachtet werden kann, liegen im sauren Bereich von 0,5 bis 5,0, bevorzugt von 0,6 bis 3,5 und besonders bevorzugt von 0,8 bis 2,0. Das anwendungsbereite Entfärbemittel sollte daher auf pH-Werte von 0,5 bis 5,0, bevorzugt von 0,6 bis 3,5 und besonders bevorzugt von 0,8 bis 2,0 eingestellt werden.

Zum Zwecke der Lagerung wird das Entfärbemittel jedoch bevorzugt auf einen alkalischen pH-Wert eingestellt, da die Verbindungen der Formeln (I) und (II) bei alkalischen pH-Werten eine bessere Stabiltität besitzen. Aus diesen Gründen ist es von Vorteil, das für die Lagerung alkalisch eingestellte Entfärbemittel kurz vor der Anwendung auf einen

sauren pH-Wert zu bringen. Kurz vor der Anwendung sollte der pH-Wert des zuvor alkalisch eingestellten Mittels daher abgesenkt werden. Die Ansäuerung des zuvor alkalisch eingestellten Entfärbemittels kurz vor der Anwendung kann durch Vermischen von zwei verschiedenen Mitteln realisiert werden, wobei das zuvor beschriebene alkalisch eingestellte erste Mittel mit einem weiteren Mittel vermischt wird, welches eine oder mehrere Säuren enthält.

Die zwei zur Herstellung des anwendungsbereiten Mittels notwendigen Komponenten werden dem Anwender zweckmäßigerweise in Form eines Kits (d.h. in Form einer Mehr-Komponenten-Verpackungseinheit) zur Verfügung gestellt, welcher mindestens zwei getrennt voneinander konfektionierte Zubereitungen (A) und (B) umfasst. Die erste Zubereitung (A) beinhaltet hierbei das bzw. die Reduktionsmittel der Formel (I) (sowie ggf. die Verbindungen der Formel (II)), wohingegen die zweite Komponente (B) die zur Absenkung des pH-Wertes notendigen Säuren umfasst. Das anwendungsbereite Entfärbemittel wird dann durch Vermischen der Zubereitungen (A) und (B) hergestellt.

Ein weiterer Gegenstand der vorliegenden Erfindung ist daher eine Mehrkomponenten-Verpackungseinheit (Kit-of-parts) zur reduktiven Entfärbung von gefärbten keratinischen Fasern, umfassend mindestens zwei getrennt voneinander konfektionierte Zubereitungen (A) und (B), wobei

- die erste Zubereitung (A) in einem kosmetischen Träger mindestens eine Verbindung der Formel (I) sowie mindestens eine Verbindung der Formel (II) enhält, wie sie zuvor bei Beschreibung des ersten Erfindungsgegenstand detailliert offenbart wurden, und
- die zweite Zubereitung (B) - bezogen auf das Gesamtgewicht der Zubereitung (B) - einen Wassergehalt von 5,0 bis 99,0 Gew.-%besitzt, mindestens eine anorganische und/oder eine organische Säure enthält und einen pH-Wert im Bereich von 0 bis 4,5, bevorzugt von 0 bis 3,5 besitzt.

Als Säuren können beispielsweise eine oder mehrere Säuren aus der Gruppe Zitronensäure, Weinsäure, Äpfelsäure, Milchsäure, Essigsäure, Schwefelsäure, Salzsäure, Phosphorsäure, Methansulfonsäure, Benzoesäure, Malonsäure, Oxalsäure, Brenztraubensäure, Oxalessigsäure (Oxobutandisäure) und/oder 1-Hydroxyethan-1,1-diphosphonsäure eingesetzt werden.

In einer besonders bevorzugten Ausführungsform ist eine erfindungsgemäße Mehrkomponenten-Verpackungseinheit (Kit-of-parts) dadurch gekennzeichnet, dass die zweite Zubereitung (B) mindestens eine Säure aus der Gruppe aus Zitronensäure, Weinsäure, Äpfelsäure, Milchsäure, Essigsäure, Schwefelsäure, Salzsäure, Phosphorsäure, Methansulfonsäure, Benzoesäure, Malonsäure, Oxalsäure, Brenztraubensäure, Oxalessigsäure (Oxobutandisäure) und/oder 1-Hydroxyethan-1,1-diphosphonsäure enthält.

Bei allen pH-Werten handelt es sich um pH-Werte, die bei einer Temperatur von 20 °C gemessen werden. Die Messung des pH-Wertes kann beispielsweise mit einer Glaselektrode des Typs N61 der Firma Schott erfolgen.

Die Zubereitung (A) der Mehrkomponenten-Verpackungseinheit ist in einer bevorzugten Ausführungsform eine wässrige Zubereitung mit einem Wassergehalt von 5,0 bis 99,0 Gew.-%, bevorzugt von 15,0 bis 98,0 Gew.-%, besonders bevorzugt 50 - 98 Gew.-%. Berechnungsgrundlage für den in Gew.-% angegebenen Wassergehalt ist hierbei die Gewichtsmenge an Wasser, die im Gesamtgewicht des Mittels enthalten ist. Der pH-Wert der Zubereitung (A) kann bei einem Wert von 7,5 bis 12,0, bevorzugt von 8,0 bis 11,5, weiter bevorzugt von 8,5 bis 11,0 und besonders bevorzugt von 9,0 bis 10,5 liegen.

In einer besonders bevorzugten Ausführungsform ist eine erfindungsgemäße Mehrkomponenten-Verpackungseinheit (Kit-of-parts) dadurch gekennzeichnet, dass die erste Zubereitung (A) - bezogen auf das Gesamtgewicht der Zubereitung (A) - einen Wassergehalt von 5,0 bis 99,0 Gew.-%, bevorzugt von 15,0 bis 98,0 Gew.-%, besonders bevorzugt 50 - 98 Gew.-% besitzt und einen pH-Wert von 7,5 bis 12,0, bevorzugt von 8,0 bis 11,5, weiter bevorzugt von 8,5 bis 11,0 und besonders bevorzugt von 9,0 bis 10,5 besitzt.

Die zur Einstellung des pH-Wertes der Zubereitung (A) erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Geeignete anorganische Alkalisierungsmittel sind Natriumhydroxid, Kaliumhydroxid, Natriumsilicat und Natriummetasilicat. Erfindungsgemäß einsetzbare, organische Alkalisierungsmittel können ausgewählt werden aus Monoethanolamin, 2-Amino-2-methylpropanol und Triethanolamin. Die als erfindungsgemäßes Alkalisierungsmittel einsetzbaren basischen Aminosäuren können ausgewählt werden aus der Gruppe, die gebildet wird aus Arginin, Lysin, Ornithin und Histidin.

Besonders bevorzugt werden die erfindungsgemäßen Entfärbemittel durch Einsatz eines oder mehrere Alkalisierungsmittel aus der Gruppe Natriumhydroxid, Kaliumhydroxid, Ammoniak, Monoethanolamin und/oder Arginin auf einen alkalischen pH-Wert gebracht. Ganz besonders bevorzugt werden aus dieser Gruppe Natriumhydroxid und/oder Kaliumhydroxid ausgewählt.

In einer weiteren bevorzugten Ausführungsform ist ein erfindungsgemäßes Entfärbemittel daher dadurch gekennzeichnet, dass es zusätzlich ein oder mehrere Alkalisierungsmittel ausgewählt aus der Gruppe Natriumhydroxid, Kaliumhydroxid, Ammoniak, Monoethanolamin und/oder Arginin, besonders bevorzugt ausgewählt aus der Gruppe

Natriumhydroxid und/oder Kaliumhydroxid, enthält.

Wie bereits zuvor beschrieben wird das anwendungsbereite Entfärbemittel bevorzugt durch Vermischen von zwei Zubereitungen (A) und (B) hergestellt. Prinzipiell können die Zubereitungen (A) und (B) hierbei in verschiedenen Mischungsverhältnissen, wie beispielsweise (A)/(B) von 20:1 bis 1:20, vermischt werden. Bevorzugt werden die Zubereitungen (A) und (B) in einem Mischungsverhältnis von 1:10 bis 10:1, besonders bevorzugt von 1:2 bis 2:1 miteinander vermischt. Die Mischungsverhältnisse geben hierbei das Verhältnis der Gewichtsmengen der Zubereitungen (A) und (B) zueinander an.

Die Zubereitungen (A) und (B) werden getrennt voneinander konfektioniert und können jeweils in jedem hierfür geeigneten Behältnis konfektioniert vorliegen. Geignete Behältnisse sind beispielsweise Glas- oder insbesondere Kunststoff-Flaschen, Dosen, Tuben oder auch andere geeignete Container.

Zur Herstellung der anwendungsbereiten Mischung kann beispielsweise das Mittel (A) aus Container (I) vollständig in Container (II) - der bereits das Mittel (B) enthält - überführt werden. In diesem Fall wird die Größe des Containers (II) so gewählt, dass der Container (II) die Gesamtmenge der Mittel (A) und (B) aufnehmen kann und auch ein Vermischen der beiden Mittel (A) und (B), z.B. durch Verschütteln oder Verrühren, zulässt.

Analog kann die Herstellung der Mischung auch durch vollständige Überführung des Mittels (B) aus Container (II) in Container (I) - der bereits das Mittel (A) enthält - erfolgen. In diesem Fall sollte die Größe des Containers (I) so gewählt werden, dass der Container (I) die Gesamtmenge der Mittel (A) und (B) aufnehmen kann und auch ein Vermischen der beiden Mittel (A) und (B), z.B. durch Verschütteln, oder Verrühren, zulässt.

Eine weitere Möglichkeit zur Herstellung der Anwendungsmischung ist die vollständige Überführung beider Mittel (A) und (B) aus den Containern (I) und (II) in ein drittes Behältnis, welches dann das Vermischen beider Mittel - z.B. durch Verschütteln, oder Verrühren - erlaubt.

Beispiel: Eine erfindungsgemäße Mehrkompenenten-Verpackungseinheit enthält

- 100 g der Zubereitung (A) in Container (I)
- 50 g der Zubereitung (B) in Conatiner (II)

Zur Herstellung der Anwendungsmischung wird die Zubereitung (B) aus Container (II) vollständig in Container (I) überführt. Die Zubereitungen (A) und (B) werden dann miteinander verschüttelt oder verrührt. Das Mischungsverhältnis der Zubereitungen (A)/(B) liegt bei einem Wert von (100 g / 50 g) = 2:1.

Die Mehrkomponentenverpackungseinheit (Kit-of-parts) kann auch noch eine weitere dritte, getrennt konfektionierte Zubereitung (C) umfassen. Dies ist insbesondere dann der Fall, wenn die Komponente (A), die das bzw. die Reduktionsmittel der Formel (I) enthält, wasserfrei konfektioniert werden soll.

In diesem Fall wird die die Reduktionsmittel der Formel (I) enthaltende Zubereitung (A) zunächst mit der wässrigen Zubereitung (C) vermischt - dieser Vermischungsvorgang gewährleistet die vollständige Lösung der Verbindungen der Formel (I) (sowie ggf. der Verbindungen der Formel (II)). Zur Herstellung der finalen Anwendungsmischung wird die Mischung der Zubereitungen (A) und (C) wird dann mit der Zubereitung (B) vermischt und hierdurch der für die Anwendung optimale pH-Wert eingestellt.

In diesem Fall ist eine besonders bevorzugte Mehrkomponenten-Verpackungseinheit (Kit-of-parts) dadurch gekennzeichnet, dass

- die erste Zubereitung (A) einen Wassergehalt von 0 bis 5,0 Gew.-%, bevorzugt von 0 bis 2,5 Gew.-%, weiter bevorzugt von 0 bis 1,0 Gew.-% und besonders bevorzugt von 0 bis 0,1 Gew.-% besitzt und
- dass die Mehrkomponenten-Verpackungseinheit mindestens eine weitere getrennt von den Zubereitungen (A) und (B) konfektionierte Zubereitung (C) umfasst, wobei
- die dritte Zubereitung (C) - bezogen auf das Gesamtgewicht der Zubereitung (C) - einen Wassergehalt von 5,0 bis 99,0 Gew.-%, bevorzugt von 15,0 bis 85,0 Gew.-%, besitzt.

Die Mittel (A), (B) sowie gegebenenfalls (C) können weiterhin zusätzliche Wirk-, Hilfs- und Zusatzstoffe enthalten, um die Entfärbeleistung zu verbessern und weitere gewünschte Eigenschaften der Mittel einzustellen. Beispielsweise kann eines oder mehrere der Mittel zusätzlich nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere,

Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, $N_2O$, Dimethylether, $CO_2$ und Luft. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Bezüglich weiterer bevorzugter Ausführungsformen der erfindungsgemäßen Mehrkomponenten-Verpackungseinheit (Kit-of-Parts) gilt mutatis mutandis das zu den erfindungsgemäßen Verfahren Gesagte.

Die erfindungsgemäßen, zuvor beschriebenen Mehrkomponenten-Verpackungseinheiten (Kit-of-Parts) lassen sich in Verfahren zur Färbung und reduktiven Entfärbung von keratinischen Fasern, insbesondere menschlichen Haaren, einsetzen.

Bevorzugt ist daher ein Verfahren zur Färbung und reduktiven Entfärbung von keratinischen Fasern, insbesondere menschlichen Haaren, umfassend die folgenden Schritte in der angegebenen Reihenfolge

(I) Applizierung eines kosmetischen Färbemittels, das mindestens einen direktziehenden Farbstoff und/oder mindestens ein Oxidationsfarbstoffvorprodukt enthält, auf die keratinischen Fasern
(II) Einwirkenlassen des Färbemittels für einen Zeitraum von 5 bis 60 Minuten
(III) Ausspülen des Färbemittels
(IV) Applizierung eines Entfärbemittels, wie es im Detail bei Beschreibung des ersten Erfindungsgegenstandes offenbart ist,
(V) Einwirkenlassen des Entfärbemittels bei 20 bis 45 °C für einen Zeitraum von 5 bis 60 Minuten, bevorzugt von 10 bis 55 Minuten, weiter bevorzugt von 15 bis 55 Minuten und besonders bevorzugt von 20 bis 50 Minuten,
(VI) Ausspülen des Entfärbemittels,
(VII) gegebenenfalls Applizierung eines Nachbehandlungsmittels auf die keratinischen Fasern, wobei das Nachbehandlungsmittel mindestens ein Tensid aus der Gruppe der anionischen, kationischen, nichtionischen amphoteren und/oder zwitterionischen Tenside enthält.

Die Schritte (I), (II) und (III) des Verfahrens stellen den Färbevorgang der Keratinfasern dar und werden demzufolge in direkter zeitlicher Abfolge hintereinander ausgeführt. Für die Abfolge der Schritte (III) und (IV) besteht prinzipiell keine zeitliche Limitierung. So kann Schritt (IV) Stunden, Tage oder auch beispielsweise bis zu sechs Wochen nach Beendigung des Schrittes (III) erfolgen.

Das Verfahren soll jedoch das unerwünschte Farbergebnis des Färbevorgangs der Schritte (I) bis (III) entfernen, daher versteht es sich von selbst, dass die Entfärbung nur dann vorgenommen werden kann, wenn die gefärbten Fasern auch noch das unerwünschtes Farbergebnis zeigen. Wurden die Keratinfasern beispielsweise mit direktziehenden Farbstoffen gefärbt und hat sich diese Färbung nach 2 Wochen schon komplett ausgewaschen, so ist ein im Anschluss daran stattfindender Entfärbevorgang weder notwendig noch erfindungsgemäß.

In Schritt (IV) des erfindungsgemäßen Verfahrens wird ein anwendungsbereites Entfärbemittel auf die keratinischen Fasern appliziert.

Bei diesem anwendungsbereiten Entfärbemittel kann es sich um ein Einkomponenten-Mittel handeln, das direkt aus dem Behältnis, in dem es konfektioniert wurde, auf die katinischen Fasern aufgetragen wird. Wie zuvor beschrieben ist es aus Stabilitätsgründen jedoch bevorzugt, das anwendungsbereite Entfärbemittel erst kurz vor der Anwendung durch Vermischen von (mindestens) zwei getrennt voneinander konfektionierten Zubereitungen (A) und (B) herzustellen. Die Herstellung des anwendungsbereiten Entfärbemittels kann beispielsweise durch Vermischen der Zubereitungen einer Mehrkomponenten-Verpackungs-Einheit (Kit-of-Parts) erfolgen, wie sie im Detail bei Beschreibung des zweiten Erfindungsgegenstands offenbart wurde.

Die Schritte (IV), (V) und (VI) des Verfahrens stellen den Entfärbevorgang der Keratinfasern dar und werden demzufolge wieder in direkter zeitlicher Abfolge hintereinander ausgeführt.

Schritt (VII) des Verfahrens, d.h. die Applizierung eines Nachbehandlungsmittels, ist optional. Für die Abfolge der Schritte (VI) und des optionalen Schrittes (VII) besteht wieder keine zeitliche Limitierung.

Es ist jedoch von Vorteil, wenn die Nachbehandlung des Schrittes (VII) maximal zwei Tage nach Abschluss des Schrittes (VI) erfolgt. Der Nachbehandlungsschritt (VII) kann auch öfters als einmal wiederholt werden, beispielsweise wenn es sich bei dem Nachbehandlungsmittel um ein Shampoo handelt.

Besonders bevorzugt ist auch ein Verfahren zur Färbung und reduktiven Entfärbung von keratinischen Fasern, insbesondere menschlichen Haaren, umfassend die folgenden Schritte in der angegebenen Reihenfolge

(I) Applizierung eines kosmetischen Färbemittels, das mindestens einen direktziehenden Farbstoff und/oder mindestens ein Oxidationsfarbstoffvorprodukt enthält, auf die keratinischen Fasern
(II) Einwirkenlassen des Färbemittels für einen Zeitraum von 5 bis 60 Minuten
(III) Ausspülen des Färbemittels
(IV) Applizierung eines Entfärbemittels, wie es im Detail bei Beschreibung des ersten Erfindungsgegenstandes offenbart ist,
(V) Einwirkenlassen des Entfärbemittels bei 20 bis 45 °C für einen Zeitraum von 5 bis 120 Minuten, bevorzugt von 10 bis 100 Minuten, weiter bevorzugt von 15 bis 90 Minuten und besonders bevorzugt von 20 bis 60 Minuten,
(VI) Ausspülen des Entfärbemittels,
(VII) Applizierung eines Nachbehandlungsmittels auf die keratinischen Fasern, wobei das Nachbehandlungsmittel mindestens ein Tensid aus der Gruppe der anionischen, kationischen, amphoteren und/oder zwitterionischen Tenside enthält.

Bezüglich weiterer bevorzugter Ausführungsformen der erfindungsgemäßen Verfahren gilt mutatis mutandis das zu den erfindungsgemäßen Mitteln und zu den erfindungsgemäßen Mehrkomponenten-Verpackungseinheiten (Kit-of-Parts) Gesagte.

## Beispiele

### 1.1. Färbung

Es wurden die folgenden Formulierungen hergestellt (alle Angaben in Gew.-%):

Färbecreme (F1)

| Rohstoff | Gew.% |
|---|---|
| Cetearylalkohol | 6,6 |
| C12-C18 Fettalkohole | 2,4 |
| Ceteareth-20 | 0,6 |
| Ceteareth-12 | 0,6 |
| Plantacare 1200 UP (Laurylglucoside, 50 - 53 %ige wässrige Lösung) | 2,0 |
| Natrium Laureth-6 Carboxylat (21 %ige wässrige Lösung) | 10,0 |
| Natriummyreth Sulfat (68 - 73 %ige wässrige Lösung) | 2,8 |
| Sodium acrylate, trimethylammoniopropylacrylamide chloride copolymer (19-21 %ige wässrige Lösung) | 3,8 |
| Natriumhydroxid | 0,26 |
| p-Toluylendiamin, Sulfat | 0,48 |
| m-Aminophenol | 0,02 |
| 4-Chlorresorcin | 0,09 |
| 2-Methylresorcin | 0,04 |
| Resorcin | 0,12 |
| Ammniumsulfat | 0,71 |

(fortgesetzt)

| Rohstoff | Gew.% |
|---|---|
| Natriumsulfit | 0,4 |
| Ascorbinsäure | 0,1 |
| 1-Hydroxyethan-1,1-diphosphonsäure (60 %ige wässrige Lösung) | 0,2 |
| Natriumwasserglas | 0,5 |
| L-Serin | 1,0 |
| Ammoniak (25 %ige wässrige Lösung) | 6,7 |
| Wasser | ad 100 |

Färbecreme (F2)

| Rohstoff | Gew.% |
|---|---|
| Cetearylalkohol | 8,5 |
| C12-C18 Fettalkohole | 2,4 |
| Ceteareth-20 | 0,6 |
| Ceteareth-12 | 0,6 |
| Plantacare 1200 UP (Laurylglucoside, 50 - 53 %ige wässrige Lösung) | 2,0 |
| Natrium Laureth-6 Carboxylat (21 %ige wässrige Lösung) | 10,0 |
| Natriummyreth Sulfat (68 - 73 %ige wässrige Lösung) | 2,8 |
| Sodium acrylate, trimethylammoniopropylacrylamide chloride copolymer (19 - 21 %ige wässrige Lösung) | 3,8 |
| Kaliumhydroxid | 0,83 |
| p-Toluylendiamin, Sulfat | 0,89 |
| m-Aminophenol | 0,04 |
| 2-Methylresorcin | 0,10 |
| Resorcin | 0,17 |
| 4-Chlorresorcin | 0,08 |
| 2-Amino-3-hydroxypyridin | 0,03 |
| 2,7-Dihydroxynaphthalin | 0,09 |
| Glycin | 1,0 |
| Natriumsulfit | 0,4 |
| Ascorbinsäure | 0,1 |
| 1-Hydroxyethan-1,1-diphosphonsäure (60 %ige wässrige Lösung) | 0,2 |
| Natriumwasserglas | 0,5 |
| Marulaöl | 0,6 |
| Monoethanolamin | 5,0 |
| Wasser | Ad 100 |

Oxidationsmittel (Ox)

| Rohstoff | Gew.% |
|---|---|
| Natriumbenzoat | 0,04 |
| Dipicolinsäure | 0,1 |
| Dinatriumpyrophosphat | 0,1 |
| Kaliumhydroxid | 0,09 |
| 1,2-Propylenglycol | 1,0 |
| 1-Hydroxyethan-1,1-diphosphonsäure (60 %ige wässrige Lösung) | 0,25 |
| Paraffinum Liquidum | 0,30 |
| Steartrimoniumchlorid | 0,39 |
| Cetearylalkohol | 3,4 |
| Ceteareth-20 | 1,0 |
| Wasserstoffperoxid (50 %ige wässrige Lösung) | 12,0 |

Haarsträhnen (Kerling Euronaturhaar weiß) wurden farbmetrisch vermessen, es wurde jeweils der L-Wert (als Maß für die Helligkeit der Haarsträhne) bestimmt.

Dann wurden die Farbcremes (F1 und F2) und das Oxidationsmittel (Ox) jeweils im Mengenverhältnis 1:1 miteinander vermischt und auf Haarsträhnen (Kerling Euronaturhaar weiß) appliziert. Das Gewichtsverhältnis Anwendungsmischung : Haar betrug 4:1, die Einwirkzeit betrug 30 Minuten bei einer Temperatur von 32 Grad Celsius. Anschließend wurden die Strähnen mit Wasser gespült, getrocknet und mindestens 24 Stunden bei Raumtemperatur ruhen gelassen.

Danach wurden die Haarsträhnen erneut farbmetrisch vermessen, es wurde wieder der L-Wert (als Maß für die Helligkeit der Haarsträhne) bestimmt.

Die Strähnen waren in einem dunkelblonden Farbton (F1 + OX) bzw. in einem mokkabraunen Farbton (F2 + Ox) gefärbt.

1.2. Entfärbung

Es wurden die folgenden Entfärbemittel hergestellt (alle Angaben in Gew.-% Aktivsubstanz):

Zubereitung (A)

| Rohstoff | Gew.% |
|---|---|
| Xanthan Gum | 0,8 |
| Propylenglycol | 1,6 |
| Cocoamidopropylbetain (40 %ige wässrige Lösung) | 4,0 |
| Cyclanon Eco (wässrige Lösung der Verbindungen $N(CH_2SO_2Na)_3$ und $N(CH_2SO_3Na)_3$ im Molverhältnis 1:1) | 20,0 |
| Wasser | ad 100 |
| pH-Wert | 9,0 - 10,5 |

Zubereitung (B)

| Rohstoff | Gew.% |
|---|---|
| Xanthan Gum | 0,9 |
| Propylenglycol | 1,8 |
| Cocoamidopropylbetain (40 %ige wässrige Lösung) | 4,4 |

(fortgesetzt)

| Rohstoff | Gew.% |
|---|---|
| Schwefelsäure (20 %ige wässrige Lösung) | 12,0 |
| Wasser | ad 100 |
| pH-Wert | 0,5 |

Die Zubereitungen (A) und (B) wurden im Mengenverhältnis 1:1 miteinander vermischt und auf die zuvor gefärbten Haarsträhnen aufgetragen.

Das Gewichtsverhältnis Anwendungsmischung : Haar betrug 4:1, die Einwirkzeit betrug 30 Minuten bei einer Temperatur von 32 Grad Celsius. Anschließend wurden die Strähnen mit Wasser gespült, getrocknet und mindestens 24 Stunden bei Raumtemperatur ruhen gelassen.

Danach wurden die Haarsträhnen erneut farbmetrisch vermessen, es wurde wieder der L-Wert (als Maß für die Helligkeit der Haarsträhne) bestimmt.

Die Beurteilung des Entfärbeergebnisses erfolgte anhand der Bestimmung des ΔL-Wertes

$$\Delta L = L(\text{nach der Entfärbung}) - L(\text{vor der Entfärbung})$$

Je höher der ΔL-Wert ist, desto besser wurden die gefärbten Haarsträhnen entfärbt.

| F1 + OX | L-Wert | ΔL-Wert |
|---|---|---|
| coloriertes Haar | 33,1 | 23,8 |
| entfärbtes Haar | 56,9 | |

| F2 + OX | L-Wert | ΔL-Wert |
|---|---|---|
| coloriertes Haar | 25,7 | 25,5 |
| entfärbtes Haar | 51,2 | |

Sowohl die mit F1+OX gefärbte Strähne als auch die mit F2+OX gefärbte Strähne war nach Anwendung des erfindungsgemäßen Entfärbemittels (Mischung der Zubereitungen (A) und (B)) in einem signifikanten Ausmaß entfärbt.

Weitere Formulierungsbeispiele

Zubereitung (C)

| Rohstoff | Gew.% |
|---|---|
| Xanthan Gum | 0,8 |
| Propylenglycol | 1,6 |
| Cocoamidopropylbetain (40 %ige wässrige Lösung) | 4,0 |
| Cyclanon Eco (wässrige Lösung der Verbindungen $N(CH_2SO_2Na)_3$ und $N(CH_2SO_3Na)_3$ im Molverhältnis 1:1) | 20,0 |
| Wasser | ad 100 |
| pH-Wert | 9,0 - 10,5 |

Zubereitung (D)

| Rohstoff | Gew.% |
|---|---|
| Cetearylalkohol | 2,66 |
| PEG-40 Castor Oil | 0,50 |
| Natriumcetearylsulfat | 0,17 |
| Hydroxyethan-1,1-disposphonsäure (1-Etidronsäure) | ad pH 0,5 |
| Wasser (dest.) | ad 100 |

Die Zubereitungen (C) und (D) wurden im Mengenverhältnis 1:1 miteinander vermischt und auf zuvor mit dem Färbemittel (F1+OX) gefärbte Haarsträhnen aufgetragen. Das Gewichtsverhältnis Anwendungsmischung (Entfärbemittel) : Haar betrug 4:1, die Einwirkzeit betrug 30 Minuten bei einer Temperatur von 32 Grad Celsius. Anschließend wurden die Strähnen mit Wasser gespült, getrocknet und mindestens 24 Stunden bei Raumtemperatur ruhen gelassen. Die Haare wurden in einem signifikanten Ausmaß entfärbt.

## Patentansprüche

**1.** Verfahren zur Färbung und reduktiven Entfärbung von keratinischen Fasern, insbesondere menschlichen Haaren, umfassend die folgenden Schritte in der angegebenen Reihenfolge

(I) Applizierung eines kosmetischen Färbemittels, das mindestens einen direktziehenden Farbstoff und/oder mindestens ein Oxidationsfarbstoffvorprodukt enthält, auf die keratinischen Fasern
(II) Einwirkenlassen des Färbemittels für einen Zeitraum von 5 bis 60 Minuten
(III) Ausspülen des Färbemittels
(IV) Applizierung eines Entfärbemittels auf die keratinischen Fasern,
(V) Einwirkenlassen des Entfärbemittels bei 20 bis 45 °C für einen Zeitraum von 5 bis 60 Minuten,
(VI) Ausspülen des Entfärbemittels,

wobei das Entfärbemittel ein Mittel zur reduktiven Entfärbung von gefärbten keratinischen Fasern, insbesondere menschlichen Haaren, ist, enthaltend in einem kosmetischen Träger

(a) mindestens eine Verbindung der Formel (I)

$$A[(CR^1R^2)SO_2M]_q \qquad (I)$$

in der

A für $N(R^3)_{3-q}$ steht
$R^1$, $R^2$ unabhängig voneinander für ein Wasserstoffatom oder für eine $C_1$-$C_6$-Alkylgruppe stehen,
$R^3$ identische oder verschiedene Reste ausgewählt aus der Gruppe aus einem Wasserstoffatom, einer $C_1$-$C_{20}$-Alkylgruppe, einer $C_3$-$C_8$-Cycloalkylgruppe ggf. substituiert durch ein bis drei $C_1$-$C_4$-Alkylreste,
M für identische oder verschiedene Reste ausgewählt aus einem Wasserstoffatom oder einem Äquivalent eines Alkali-, Erdalkali- oder Metallions, bevorzugt für Natrium, Kalium, ½ Magnesium, ½ Calcium, ½ Zink, oder für ein Ammoniumion ($NH_4^+$) steht,
q für die Zahlen 2 oder 3 steht,

und
(b) mindestens eine Verbindung der Formel (II)

$$A[(CR^1R^2)SO_3M]_q \qquad (II)$$

worin A, $R^1$, $R^2$, $R^3$, M, und q dieselbe allgemeine Bedeutung wie in Formel (I) besitzen, wobei die Auswahl dieser Variablen im konkreten Einzelfall für die Verbindungen der Formeln (I) und (II) nicht gleich sein muss.

**2.** Verfahren nach Anspruch 1, **dadurch gekennzeichnet, dass** das Entfärbemittel (a) mindestens eine Verbindung der Formel (I) enthält, in der

q für die Zahl 3 steht.

**3.** Verfahren nach einem der Ansprüche 1 bis 2, **dadurch gekennzeichnet, dass** das Entfärbemittel (a) mindestens eine Verbindung der Formel (I) enthält, in der

R1, R2 unabhängig voneinander für ein Wasserstoffatom oder für eine Methylgruppe, bevorzugt für ein Wasserstoffatom, stehen.

**4.** Verfahren nach einem der Ansprüche 1 bis 3, **dadurch gekennzeichnet, dass** das Entfärbemittel (a) mindestens eine Verbindung der Formel (I) enthält, in der

M für identische oder verschiedene Reste ausgewählt aus einem Wasserstoffatom oder einem Äquivalent eines Alkali-, Erdalkali- oder Metallions aus der Gruppe aus Natrium, Kalium und ½ Zink steht.

**5.** Verfahren nach einem der Ansprüche 1 bis 4, **dadurch gekennzeichnet, dass** das Entfärbemittel (a) mindestens eine Verbindung der Formel (I) enthält, die ausgewählt ist aus der Gruppe aus

- $HN(CH_2SO_2Na)_2$, Dinatrium [(sulfinatomethyl)amino]methanesulfinat
- $HN(CH_2SO_2K)_2$, Dikalium [(sulfinatomethyl)amino]methanesulfinat
- $HN(CH_2SO_2H)_2$, [(Sulfinomethyl)amino]methansulfininsäure
- $N(CH_2SO_2Na)_3$, Trinatrium [bis(sulfinatomethyl)amino]methansulfinat
- $N(CH_2SO_2K)_3$, Trikalium [bis(sulfinatomethyl)amino]methansulfinat
- $N(CH_2SO_2H)_3$, [Bis(sulfinomethyl)amino]methansulfinsäure
- $HN(CH(CH_3)SO_2Na)_2$, Dinatrium 1-[(1-sulfinatoethyl)amino]ethan-1-sulfinat
- $HN(CH(CH_3)SO_2K)_2$, Dikalium 1-[(1-sulfinatoethyl)amino]ethan-1-sulfinat
- $HN(CH(CH_3)SO_2H)_2$, 1-[(1-Sulfinoethyl)amino]ethan-1-sulfinsäure
- $N(CH(CH_3)SO_2Na)_3$, Trinatrium 1-[Bis(1-sulfinatoethyl)amino]ethan-1-sulfinat
- $N(CH(CH_3)SO_2K)_3$, Trikalium 1-[Bis(1-sulfinatoethyl)amino]ethan-1-sulinat und/oder
- $N(CH(CH_3)SO_2H)_3$, 1-[Bis(1-sulfinoethyl)amino]ethan-1-sulfinsäure.

**6.** Verfahren nach einem der Ansprüche 1 bis 5, **dadurch gekennzeichnet, dass** das Entfärbemittel (b) mindestens eine Verbindung der Formel (II) enthält, die ausgewählt ist aus der Gruppe aus

- $HN(CH_2SO_3Na)_2$, Dinatrium [(sulfonatomethyl)amino]methansulfonat
- $HN(CH_2SO_3K)_2$, Dikalium [(sulfonatomethyl)amino]methansulfonat
- $HN(CH_2SO_3H)_2$, [(Sulfomethyl)amino]methansulfonsäure
- $N(CH_2SO_3Na)_3$, Trinatrium [bis(sulfonatomethyl)amino]methansulfonat
- $N(CH_2SO_3K)_3$, Trikalium [bis(sulfonatomethyl)amino]methansulfonat
- $N(CH_2SO_3H)_3$, [Bis(sulfomethyl)amino]methansulfonsäure
- $HN(CH(CH_3)SO_3Na)_2$, Dinatrium 1-[(1-sulfonatoethyl)amino]ethan-1-sulfonat
- $HN(CH(CH_3)SO_3K)_2$, Dikalium 1-[(1-sulfonatoethyl)amino]ethan-1-sulfonat
- $HN(CH(CH_3)SO_3H)_2$, 1-[(1-Sulfoethyl)amino]ethan-1-sulfonsäure.
- $N(CH(CH_3)SO_3Na)_3$, Trinatrium 1-[bis(1-sulfonatoethyl)amino]ethan-1-sulfonat
- $N(CH(CH_3)SO_3K)_3$, Trikalium 1-[bis(1-sulfonatoethyl)amino]ethan-1-sulfonat und/oder
- $N(CH(CH_3)SO_3H)_3$, 1-[Bis(1-sulfoethyl)amino]ethan-1-sulfonsäure

**7.** Verfahren nach einem der Ansprüche 1 bis 6, **dadurch gekennzeichnet, dass** das Entfärbemittel - bezogen auf das Gesamtgewicht des Mittels - eine oder mehrere Verbindungen der Formel (I) in einer Gesamtmenge von 0,1 bis 30,0 Gew.-%, bevorzugt von 0,2 bis 20,0 Gew.-%, weiter bevorzugt von 0,3 bis 10,0 Gew.-% und ganz besonders bevorzugt von 0,5 bis 6,0 Gew.-% enthält.

**8.** Verfahren nach einem der Ansprüche 1 bis 7, **dadurch gekennzeichnet, dass** das Entfärbemittel zusätzlich mindestens ein Tensid aus der Gruppe der anionischen, amphoteren und/oder zwitterionischen, nichtionischen und/oder kationischen Tenside enthält.

9. Verfahren nach einem der Ansprüche 1 bis 8, **dadurch gekennzeichnet, dass** das Entfärbemittel zusätzlich mindestens ein Verdickungsmittel enthält, das ausgewählt ist aus der Gruppe der anionischen, synthetischen Polymere, der kationischen, synthetischen Polymere, der nichtionischen Guargums, der Skleroglucangums, der Xanthangums, Gummi arabicum, Carrageen-Gummi, Agar-Agar, Johannisbrotkernmehl, der Pektine, der Alginate, der Stärke, der Cellulosen und Cellulosederivate und/oder der nichtionischen, synthetischen Polymere.

10. Mehrkomponenten-Verpackungseinheit (Kit-of-parts) zur reduktiven Entfärbung von gefärbten keratinischen Fasern, umfassend mindestens zwei getrennt voneinander konfektionierte Zubereitungen (A) und (B), wobei

    - die erste Zubereitung (A) in einem kosmetischen Träger mindestens eine Verbindung der Formel (I) sowie mindestens eine Verbindung der Formel (II) enthält, wie sie in einem der Ansprüche 1 bis 6 definiert sind, und
    - die zweite Zubereitung (B) - bezogen auf das Gesamtgewicht der Zubereitung (B) - einen Wassergehalt von 5,0 bis 99,0 Gew.-% besitzt, mindestens eine anorganische und/oder eine organische Säure enthält und einen pH-Wert im Bereich von 0 bis 4,5, bevorzugt von 0 bis 3,5 besitzt.

11. Mehrkomponenten-Verpackungseinheit (Kit-of-parts) nach Anspruch 10, **dadurch gekennzeichnet, dass** die zweite Zubereitung (B) mindestens eine Säure aus der Gruppe aus Zitronensäure, Weinsäure, Äpfelsäure, Milchsäure, Essigsäure, Schwefelsäure, Salzsäure, Phosphorsäure, Methansulfonsäure, Benzoesäure, Malonsäure, Oxalsäure, Brenztraubensäure, Oxalessigsäure (Oxobutandisäure) und/oder 1-Hydroxyethan-1,1-diphosphonsäure enthält.

12. Mehrkomponenten-Verpackungseinheit (Kit-of-parts) nach einem der Ansprüche 10 bis 11, **dadurch gekennzeichnet, dass** die erste Zubereitung (A) - bezogen auf das Gesamtgewicht der Zubereitung (A) - einen Wassergehalt von 5,0 bis 99,0 Gew.-%, bevorzugt von 15,0 bis 98,0 Gew.-%, besonders bevorzugt 50 - 98 Gew.-% besitzt und einen pH-Wert von 7,5 bis 12,0, bevorzugt von 8,0 bis 11,5, weiter bevorzugt von 8,5 bis 11,0 und besonders bevorzugt von 9,0 bis 10,5 besitzt.

13. Mehrkomponenten-Verpackungseinheit (Kit-of-parts) nach einem der Ansprüche 10 bis 11, **dadurch gekennzeichnet, dass**

    - die erste Zubereitung (A) einen Wassergehalt von 0 bis 5,0 Gew.-%, bevorzugt von 0 bis 2,5 Gew.-%, weiter bevorzugt von 0 bis 1,0 Gew.-% und besonders bevorzugt von 0 bis 0,1 Gew.-% besitzt und dass die Mehrkomponenten-Verpackungseinheit mindestens eine weitere getrennt von den Zubereitungen (A) und (B) konfektionierte Zubereitung (C) umfasst, wobei
    - die dritte Zubereitung (C) - bezogen auf das Gesamtgewicht der Zubereitung (C) - einen Wassergehalt von 5,0 bis 99,0 Gew.-%, bevorzugt von 15,0 bis 85,0 Gew.-%, besitzt.

14. Verfahren nach einem der Ansprüche 1 bis 9 umfassend die folgenden Schritte in der angegebenen Reihenfolge

    (I) Applizierung eines kosmetischen Färbemittels, das mindestens einen direktziehenden Farbstoff und/oder mindestens ein Oxidationsfarbstoffvorprodukt enthält, auf die keratinischen Fasern
    (II) Einwirkenlassen des Färbemittels für einen Zeitraum von 5 bis 60 Minuten
    (III) Ausspülen des Färbemittels
    (IV) Applizierung eines Entfärbemittels auf die keratinischen Fasern,
    (V) Einwirkenlassen des Entfärbemittels bei 20 bis 45 °C für einen Zeitraum von 10 bis 55 Minuten, weiter bevorzugt von 15 bis 55 Minuten und besonders bevorzugt von 20 bis 50 Minuten,
    (VI) Ausspülen des Entfärbemittels,
    (VII) gegebenenfalls Applizierung eines Nachbehandlungsmittels auf die keratinischen Fasern, wobei das Nachbehandlungsmittel mindestens ein Tensid aus der Gruppe der anionischen, kationischen, nichtionischen amphoteren und/oder zwitterionischen Tenside enthält.

## Claims

1. A method for dyeing and reductively decolorizing keratin fibers, in particular human hair, comprising the following steps in the sequence indicated

    (I) applying a cosmetic colorant containing at least one direct dye and/or at least one oxidation dye precursor to the keratin fibers

(II) allowing the colorant to act for a period of 5 to 60 minutes
(III) rinsing out the colorant
(IV) applying a decolorizing agent to the keratin fibers,
(V) allowing the decolorizing agent to act at 20 to 45 °C for a period of 5 to 60 minutes,
(VI) rinsing out the decolorizing agent,

wherein the decolorizing agent is an agent for reductively decolorizing dyed keratin fibers, in particular human hair, containing, in a cosmetic carrier,

(a) at least one compound of formula (I)

$$A[(CR^1R^2)SO_2M]_q \qquad (I)$$

in which

A represents $N(R^3)_{3-q}$
$R^1$, $R^2$ represent, independently of one another, a hydrogen atom or a $C_1$-$C_6$ alkyl group,
$R^3$ represents identical or different functional groups selected from the group consisting of a hydrogen atom, a $C_1$-$C_{20}$ alkyl group, a $C_3$-$C_8$ cycloalkyl group optionally substituted by one to three $C_1$-$C_4$ alkyl functional groups,
M represents identical or different functional groups selected from a hydrogen atom or an equivalent of an alkali, alkaline-earth or metal ion, preferably sodium, potassium, ½ magnesium, ½ calcium, ½ zinc, or represents an ammonium ion ($NH_4^+$),
q represents the number 2 or 3,

and
(b) at least one compound of formula (II)

$$A[(CR^1R^2)SO_3M]_q \qquad (II)$$

in which A, $R^1$, $R^2$, $R^3$, M, and q have the same general meaning as in formula (I), wherein the selection of these variables in each specific case does not need to be the same for the compounds of formulas (I) and (II).

**2.** The method according to claim 1, **characterized in that** the decolorizing agent (a) contains at least one compound of formula (I), in which

q represents the number 3.

**3.** The method according to one of claims 1 to 2, **characterized in that** the decolorizing agent (a) contains at least one compound of formula (I), in which

R1, R2 represent, independently of one another, a hydrogen atom or a methyl group, preferably a hydrogen atom.

**4.** The method according to one of claims 1 to 3, **characterized in that** the decolorizing agent (a) contains at least one compound of formula (I), in which

M represents identical or different functional groups selected from a hydrogen atom or an equivalent of an alkali, alkaline-earth or metal ion from the group consisting of sodium, potassium and ½ zinc.

**5.** The method according to one of claims 1 to 4, **characterized in that** the decolorizing agent (a) contains at least one compound of formula (I), which is selected from the group consisting of

- $HN(CH_2SO_2Na)_2$, disodium [(sulfinatomethyl)amino]methanesulfinate
- $HN(CH_2SO_2K)_2$, dipotassium [(sulfinatomethyl)amino]methanesulfinate
- $HN(CH_2SO_2H)_2$, [(sulfinomethyl)amino]methanesulfinic acid
- $N(CH_2SO_2Na)_3$, trisodium [bis(sulfinatomethyl)amino]methanesulfinate
- $N(CH_2SO_2K)_3$, tripotassium [bis(sulfinatomethyl)amino]methanesulfinate
- $N(CH_2SO_2H)_3$, [bis(sulfinomethyl)amino]methanesulfinic acid

- $HN(CH(CH_3)SO_2Na)_2$, disodium 1-[(1-sulfinatoethyl)amino]ethane-1-sulfinate
- $HN(CH(CH_3)SO_2K)_2$, dipotassium 1-[(1-sulfinatoethyl)amino]ethane-1-sulfinate
- $HN(CH(CH_3)SO_2H)_2$, 1-[(1-sulfinoethyl)amino]ethane-1-sulfinic acid
- $N(CH(CH_3)SO_2Na)_3$, trisodium 1-[bis(1-sulfinatoethyl)amino]ethane-1-sulfinate
- $N(CH(CH_3)SO_2K)_3$, tripotassium 1-[bis(1-sulfinatoethyl)amino]ethane-1-sulinate and/or
- $N(CH(CH_3)SO_2H)_3$, 1-[bis(1-sulfinoethyl)amino]ethane-1-sulfinic acid.

**6.** The method according to one of claims 1 to 5, **characterized in that** the decolorizing agent (b) contains at least one compound of formula (II), which is selected from the group consisting of

- $HN(CH_2SO_3Na)_2$, disodium [(sulfonatomethyl)amino]methanesulfonate
- $HN(CH_2SO_3K)_2$, dipotassium [(sulfonatomethyl)amino]methanesulfonate
- $HN(CH_2SO_3H)_2$, [(sulfomethyl)amino]methanesulfonic acid
- $N(CH_2SO_3Na)_3$, trisodium [bis(sulfonatomethyl)amino]methanesulfonate
- $N(CH_2SO_3K)_3$, tripotassium [bis(sulfonatomethyl)amino]methanesulfonate
- $N(CH_2SO_3H)_3$, [bis(sulfomethyl)amino]methanesulfonic acid
- $HN(CH(CH_3)SO_3Na)_2$, disodium 1-[(1-sulfonatoethyl)amino]ethane-1-sulfonate
- $HN(CH(CH_3)SO_3K)_2$, dipotassium 1-[(1-sulfonatoethyl)amino]ethane-1-sulfonate
- $HN(CH(CH_3)SO_3H)_2$, 1-[(1-sulfoethyl)amino]ethane-1-sulfonic acid
- $N(CH(CH_3)SO_3Na)_3$, trisodium 1-[bis(1-sulfonatoethyl)amino]ethane-1-sulfonate
- $N(CH(CH_3)SO_3K)_3$, tripotassium 1-[bis(1-sulfonatoethyl)amino]ethane-1-sulfonate and/or
- $N(CH(CH_3)SO_3H)_3$, 1-[bis(1-sulfoethyl)amino]ethane-1-sulfonic acid.

**7.** The method according to one of claims 1 to 6, **characterized in that** the decolorizing agent contains, based on the total weight of the agent, one or more compounds of formula (I) in a total amount of from 0.1 to 30.0 wt.%, preferably from 0.2 to 20.0 wt.%, more preferably from 0.3 to 10.0 wt.%, and very particularly preferably from 0.5 to 6.0 wt.%.

**8.** The method according to one of claims 1 to 7, **characterized in that** the decolorizing agent additionally contains at least one surfactant from the group of anionic, amphoteric and/or zwitterionic, non-ionic and/or cationic surfactants.

**9.** The method according to one of claims 1 to 8, **characterized in that** the decolorizing agent additionally contains at least one thickener selected from the group of anionic, synthetic polymers, cationic, synthetic polymers, non-ionic guar gums, scleroglucan gums, xanthan gums, arabic gum, carrageenan gum, agar agar, carob gum, pectins, alginates, starch, celluloses and cellulose derivatives and/or non-ionic, synthetic polymers.

**10.** A multicomponent packaging unit (kit-of-parts) for reductively decolorizing dyed keratin fibers, comprising at least two preparations (A) and (B) packaged separately from one another, wherein

- the first preparation (A) contains, in a cosmetic carrier, at least one compound of formula (I) and at least one compound of formula (II), as defined in one of claims 1 to 6, and
- the second preparation (B) has, based on the total weight of preparation (B), a water content of from 5.0 to 99.0 wt.%, contains at least one inorganic and/or one organic acid and has a pH in the range of from 0 to 4.5, preferably from 0 to 3.5.

**11.** The multicomponent packaging unit (kit-of-parts) according to claim 10, **characterized in that** the second preparation (B) contains at least one acid from the group consisting of citric acid, tartaric acid, malic acid, lactic acid, acetic acid, sulfuric acid, hydrochloric acid, phosphoric acid, methanesulfonic acid, benzoic acid, malonic acid, oxalic acid, pyruvic acid, oxaloacetic acid (oxobutanedioic acid) and/or 1-hydroxyethane-1,1-diphosphonic acid.

**12.** The multicomponent packaging unit (kit-of-parts) according to one of claims 10 to 11, **characterized in that** the first preparation (A) has, based on the total weight of preparation (A), a water content of from 5.0 to 99.0 wt.%, preferably from 15.0 to 98.0 wt.%, particularly preferably 50-98 wt.%, and has a pH of from 7.5 to 12.0, preferably from 8.0 to 11.5, more preferably from 8.5 to 11.0, and particularly preferably from 9.0 to 10.5.

**13.** The multicomponent packaging unit (kit-of-parts) according to one of claims 10 to 11, **characterized in that**

- the first preparation (A) has a water content of from 0 to 5.0 wt.%, preferably from 0 to 2.5 wt.%, more preferably from 0 to 1.0 wt.% and particularly preferably from 0 to 0.1 wt.%, and **in that** the multicomponent packaging

unit comprises at least one further preparation (C) packaged separately from preparations (A) and (B),
- the third preparation (C) having, based on the total weight of preparation (C), a water content of from 5.0 to 99.0 wt.%, preferably from 15.0 to 85.0 wt.%.

**14.** The method according to one of claims 1 to 9, comprising the following steps in the sequence indicated

(I) applying a cosmetic colorant containing at least one direct dye and/or at least one oxidation dye precursor to the keratin fibers
(II) allowing the colorant to act for a period of 5 to 60 minutes
(III) rinsing out the colorant
(IV) applying a decolorizing agent to the keratin fibers,
(V) allowing the decolorizing agent to act at 20 to 45 °C for a period of 10 to 55 minutes, more preferably 15 to 55 minutes, and particularly preferably 20 to 50 minutes,
(VI) rinsing out the decolorizing agent,
(VII) optionally applying a post-treatment agent to the keratin fibers, wherein the post-treatment agent contains at least one surfactant from the group of anionic, cationic, non-ionic amphoteric and/or zwitterionic surfactants.

## Revendications

**1.** Procédé de coloration et de décoloration réductrice de fibres kératiniques, en particulier de cheveux humains, comprenant les étapes suivantes, dans l'ordre indiqué, consistant à :

(I) appliquer un agent de coloration cosmétique contenant au moins un colorant direct et/ou au moins un précurseur de colorant d'oxydation sur les fibres kératiniques,
(II) laisser agir l'agent de coloration pendant une durée de 5 à 60 minutes,
(III) rincer l'agent de coloration,
(IV) appliquer un agent de décoloration sur les fibres kératiniques,
(V) laisser agir l'agent de décoloration à une température de 20 à 45 °C pendant une durée de 5 à 60 minutes,
(VI) rincer l'agent de décoloration,

l'agent de décoloration étant un agent pour la décoloration réductrice de fibres kératiniques colorées, en particulier de cheveux humains, contenant, dans un support cosmétique,

(a) au moins un composé de formule (I)

$$A[(CR^1R^2)SO_2M]_q \qquad (I)$$

dans laquelle

A représente $N(R^3)_{3-q}$
$R^1$, $R^2$ représentent indépendamment un atome d'hydrogène ou un groupe alkyle en $C_1$-$C_6$,
$R^3$ représente des radicaux identiques ou différents choisis dans le groupe constitué par un atome d'hydrogène, un groupe alkyle en $C_1$-$C_{20}$, un groupe cycloalkyle en $C_3$-$C_8$ éventuellement substitué par un à trois radicaux alkyle en $C_1$-$C_4$,
M représente des radicaux identiques ou différents choisis parmi un atome d'hydrogène ou un équivalent d'un ion alcalin, alcalino-terreux ou métallique, de préférence un ion sodium, potassium, ½ magnésium, ½ calcium, ½ zinc, ou ammonium ($NH_4^+$),
q représente les nombres 2 ou 3,

et
(b) au moins un composé de formule (II)

$$A[(CR^1R^2)SO_3M]_q \qquad (II)$$

dans laquelle A, $R^1$, $R^2$, $R^3$, M et q ont la même signification générale que dans la formule (I), le choix de ces variables ne devant pas être le même dans le cas individuel spécifique pour les composés de formules (I) et (II).

2. Procédé selon la revendication 1, **caractérisé en ce que** l'agent de décoloration (a) contient au moins un composé de formule (I), dans laquelle

   q représente le nombre 3.

3. Procédé selon l'une des revendications 1 à 2, **caractérisé en ce que** l'agent de décoloration (a) contient au moins un composé de formule (I), dans laquelle

   $R^1$ et $R^2$ représentent indépendamment un atome d'hydrogène ou un groupe méthyle, de préférence un atome d'hydrogène.

4. Procédé selon l'une des revendications 1 à 3, **caractérisé en ce que** l'agent de décoloration (a) contient au moins un composé de formule (I), dans laquelle

   M représente des radicaux identiques ou différents choisis parmi un atome d'hydrogène ou un équivalent d'un ion alcalin, alcalino-terreux ou métallique choisi dans le groupe constitué par le sodium, le potassium et le ½ zinc.

5. Procédé selon l'une des revendications 1 à 4, **caractérisé en ce que** l'agent de décoloration (a) contient au moins un composé de formule (I), choisi dans le groupe constitué par

   - $HN(CH_2SO_2Na)_2$, [(sulfinatométhyl)amino]méthanesulfinate disodique
   - $HN(CH_2SO_2K)_2$, [(sulfinatométhyl)amino]méthanesulfinate dipotassique
   - $HN(CH_2SO_2H)_2$, acide [(sulfinométhyl)amino]méthanesulfinique
   - $N(CH_2SO_2Na)_3$, [bis(sulfinatométhyl)amino]méthanesulfinate trisodique
   - $N(CH_2SO_2K)_3$, [bis(sulfinatométhyl)amino]méthanesulfinate tripotassique
   - $N(CH_2SO_2H)_3$, acide [bis(sulfinométhyl)amino]méthanesulfinique
   - $HN(CH(CH_3)SO_2Na)_2$, 1-[(1-sulfinatoéthyl)amino]éthane-1-sulfinate disodique
   - $HN(CH(CH_3)SO_2K)_2$, 1-[(1-sulfinatoéthyl)amino]éthane-1-sulfinate dipotassique
   - $HN(CH(CH_3)SO_2H)_2$, acide 1-[(1-sulfinoéthyl)amino]éthane-1-sulfinique
   - $N(CH(CH_3)SO_2Na)_3$, 1-[bis(1-sulfinatoéthyl)amino]éthane-1-sulfinate trisodique
   - $N(CH(CH_3)SO_2K)_3$, 1-[bis(1-sulfinatoéthyl)amino]éthane-1-sulinate tripotassique et/ou
   - $N(CH(CH_3)SO_2H)_3$, acide 1-[bis(1-sulfinoéthyl)amino]éthane-1-sulfinique.

6. Procédé selon l'une des revendications 1 à 5, **caractérisé en ce que** l'agent de décoloration (b) contient au moins un composé de formule (II) choisi dans le groupe constitué par

   - $HN(CH_2SO_3Na)_2$, [(sulfonatométhyl)amino]méthanesulfonate disodique
   - $HN(CH_2SO_3K)_2$, [(sulfonatométhyl)amino]méthanesulfonate dipotassique
   - $HN(CH_2SO_3H)_2$, acide [(sulfométhyl)amino]méthanesulfonique
   - $N(CH_2SO_3Na)_3$, [bis(sulfonatométhyl)amino]méthanesulfonate trisodique
   - $N(CH_2SO_3K)_3$, [bis(sulfonatométhyl)amino]méthanesulfonate tripotassique
   - $N(CH_2SO_3H)_3$, acide [bis(sulfométhyl)amino]méthanesulfonique
   - $HN(CH(CH_3)SO_3Na)_2$, 1-[(1-sulfonatoéthyl)amino]éthane-1-sulfonate disodique
   - $HN(CH(CH_3)SO_3K)_2$, 1-[(1-sulfonatoéthyl)amino]éthane-1-sulfonate dipotassique
   - $HN(CH(CH_3)SO_3H)_2$, acide 1-[(1-sulfoéthyl)amino]éthane-1-sulfonique
   - $N(CH(CH_3)SO_3Na)_3$, 1-[bis(1-sulfonatoéthyl)amino]éthane-1-sulfonate trisodique
   - $N(CH(CH_3)SO_3K)_3$, 1-[bis(1-sulfonatoéthyl)amino]éthane-1-sulfonate tripotassique et/ou
   - $N(CH(CH_3)SO_3H)_3$, acide 1-[bis(1-sulfoéthyl)amino]éthane-1-sulfonique.

7. Procédé selon l'une des revendications 1 à 6, **caractérisé en ce que** l'agent de décoloration contient, par rapport au poids total de l'agent, un ou plusieurs composés de formule (I) en une quantité totale de 0,1 à 30,0 % en poids, de préférence de 0,2 à 20,0 % en poids, de manière davantage préférée de 0,3 à 10,0 % en poids et de manière particulièrement préférée de 0,5 à 6,0 % en poids.

8. Procédé selon l'une des revendications 1 à 7, **caractérisé en ce que** l'agent de décoloration contient en outre au moins un tensioactif du groupe constitué par des tensioactifs anioniques, amphotères et/ou zwitterioniques, non ioniques et/ou cationiques.

**9.** Procédé selon l'une des revendications 1 à 8, **caractérisé en ce que** l'agent de décoloration contient en outre au moins un agent épaississant choisi dans le groupe constitué par les polymères synthétiques anioniques, les polymères synthétiques cationiques, des gommes de guar non ioniques, les gommes de scléroglucane, les gommes de xanthane, les gommes arabiques, les gommes carraghénanes, les agar-agar, la farine de caroube, les pectines, les alginates, l'amidon, les celluloses et les dérivés de cellulose et/ou des polymères synthétiques non-ioniques.

**10.** Unité d'emballage à plusieurs composants (kit de pièces) pour la décoloration réductrice de fibres kératiniques colorées, comprenant au moins deux préparations (A) et (B) confectionnées séparément l'une de l'autre,

- la première préparation (A) contenant, dans un support cosmétique, au moins un composé de formule (I) et au moins un composé de formule (II) tels que définis dans l'une des revendications 1 à 6, et
- la seconde préparation (B) présentant, par rapport au poids total de la préparation (B), une teneur en eau de 5,0 à 99,0 % en poids, contenant au moins un acide inorganique et/ou un acide organique et présentant une valeur pH comprise entre 0 et 4,5, de préférence entre 0 et 3,5.

**11.** Unité d'emballage à plusieurs composants (kit de pièces) selon la revendication 10, **caractérisée en ce que** la seconde préparation (B) contient au moins un acide du groupe constitué par l'acide citrique, l'acide tartrique, l'acide malique, l'acide lactique, l'acide acétique, l'acide sulfurique, l'acide chlorhydrique, l'acide phosphorique, l'acide méthanesulfonique, l'acide benzoïque, l'acide malonique, l'acide oxalique, l'acide pyruvique, l'acide oxalacique (acide oxobutanoïque) et/ou l'acide 1-hydroxyéthane-1,1-diphosphonique.

**12.** Unité d'emballage à plusieurs composants (kit de pièces) selon l'une des revendications 10 à 11, **caractérisée en ce que** la première préparation (A) présente, par rapport au poids total de la préparation (A), une teneur en eau de 5,0 à 99,0 % en poids, de préférence de 15,0 à 98,0 % en poids, de manière particulièrement préférée de 50 à 98 % en poids, et une valeur pH de 7,5 à 12,0, de préférence de 8,0 à 11,5, de manière davantage préférée de 8,5 à 11,0 et de manière particulièrement préférée de 9,0 à 10,5.

**13.** Unité d'emballage à plusieurs composants (kit de pièces) selon l'une des revendications 10 à 11, **caractérisée en ce que**

- la première préparation (A) présente une teneur en eau de 0 à 5,0 % en poids, de préférence de 0 à 2,5 % en poids, de manière davantage préférée de 0 à 1,0 % en poids et de manière particulièrement préférée de 0 à 0,1 % en poids, et **en ce que** l'unité d'emballage à plusieurs composants comprend au moins une autre préparation (C) confectionnée séparément des préparations (A) et (B),
- la troisième préparation (C) présentant, par rapport au poids total de la préparation (C), présentant une teneur en eau de 5,0 à 99,0 % en poids, de préférence de 15,0 à 85,0 % en poids.

**14.** Procédé selon l'une des revendications 1 à 9 comprenant les étapes suivantes, dans l'ordre indiqué, consistant à :

(I) appliquer un agent de coloration cosmétique contenant au moins un colorant direct et/ou au moins un précurseur de colorant d'oxydation sur les fibres kératiniques,
(II) laisser agir l'agent de coloration pendant une durée de 5 à 60 minutes,
(III) rincer l'agent de coloration,
(IV) appliquer un agent de décoloration sur les fibres kératiniques,
(V) laisser agir l'agent de décoloration à une température de 20 à 45 °C pendant une durée de 10 à 55 minutes, de manière davantage préférée de 15 à 55 minutes et de manière particulièrement préférée de 20 à 50 minutes,
(VI) rincer l'agent de décoloration,
(VII) appliquer éventuellement un agent de post-traitement sur les fibres kératiniques, l'agent de post-traitement contenant au moins un tensioactif du groupe des tensioactifs anioniques, cationiques, non ioniques, amphotères et/ou zwitterioniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1300136 A2
- WO 2008055756 A2
- DE 19629453 A1
- DE 102006022254 A1
- DE 102006053402 A1
- WO 2007107310 A2
- DE 102006053343 A1
- EP 0914516 B1


### In der Beschreibung aufgeführte Nicht-Patentliteratur


- **KH. SCHRADER.** Grundlagen und Rezepturen der Kosmetika. Hüthig Buch Verlag, 1989
- *CHEMICAL ABSTRACTS,* 7775-14-6
- *CHEMICAL ABSTRACTS,* 7779-86-4
- *CHEMICAL ABSTRACTS,* 14293-73-3
- *CHEMICAL ABSTRACTS,* 7757-83-7
- *CHEMICAL ABSTRACTS,* 7631-90-5
- *CHEMICAL ABSTRACTS,* 10117-38-1
- *CHEMICAL ABSTRACTS,* 7773-03-7
- *CHEMICAL ABSTRACTS,* 10196-04-0
- *CHEMICAL ABSTRACTS,* 7772-98-7
- *CHEMICAL ABSTRACTS,* 10294-66-3
- *CHEMICAL ABSTRACTS,* 7783-18-8
- *CHEMICAL ABSTRACTS,* 79-25-4
- *CHEMICAL ABSTRACTS,* 118201-33-5
- *CHEMICAL ABSTRACTS,* 68-11-1
- *CHEMICAL ABSTRACTS,* 144-62-7
- *CHEMICAL ABSTRACTS,* 328-42-7
- *CHEMICAL ABSTRACTS,* 50-81-7